(19) Europäisches Patentamt
European Patent Office
Office européen des brevets

(11) **EP 3 209 248 B1**

(12) **EUROPÄISCHE PATENTSCHRIFT**

(45) Veröffentlichungstag und Bekanntmachung des Hinweises auf die Patenterteilung:
**17.02.2021 Patentblatt 2021/07**

(21) Anmeldenummer: **15781665.3**

(22) Anmeldetag: **19.10.2015**

(51) Int Cl.:
*A61F 2/90* (2013.01)      *A61F 2/95* (2013.01)
*D04C 1/06* (2006.01)

(86) Internationale Anmeldenummer:
**PCT/EP2015/074155**

(87) Internationale Veröffentlichungsnummer:
**WO 2016/062665 (28.04.2016 Gazette 2016/17)**

(54) **STENT ZUM SCHIENEN EINER VENE UND SYSTEM ZUM SETZEN EINES STENTS**

STENT FOR SPLINTING A VEIN, AND SYSTEM FOR PUTTING IN PLACE A STENT

STENT DESTINÉ À SERVIR DE TUTEUR À UNE VEINE ET SYSTÈME POUR POSER UN STENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.10.2014 DE 102014115337**

(43) Veröffentlichungstag der Anmeldung:
**30.08.2017 Patentblatt 2017/35**

(60) Teilanmeldung:
**21151615.8**

(73) Patentinhaber:
• **Düring, Klaus**
**50226 Frechen (DE)**
• **Dlaikan-Campos, Nasib**
**52146 Würselen (DE)**

(72) Erfinder:
• **Düring, Klaus**
**50226 Frechen (DE)**
• **Dlaikan-Campos, Nasib**
**52146 Würselen (DE)**

(74) Vertreter: **HGF**
**Neumarkter Straße 18**
**81673 München (DE)**

(56) Entgegenhaltungen:
EP-A1- 2 165 684        WO-A1-96/25124
WO-A1-2006/037086       DE-A1-102007 061 931
US-A- 5 725 547         US-A1- 2005 119 722
US-A1- 2013 090 720     US-A1- 2014 277 573

**Beschreibung**

Die vorliegende Erfindung betrifft einen Stent zum Schienen einer Vene sowie ein System zum Setzen eines Stents.

Es ist bekannt, bei Gefäßverengungen und -verschlüssen Stents zu setzen, mit welchen die Gefäße offen gehalten werden. Stents werden vor allem in Arterien eingesetzt. Ein solcher Stent ist ein kurzes, rohrförmiges Teil, das aus Metall oder Kunststoff ausgebildet ist. In der Regel weist es eine gitterartige Struktur auf, die durch (Laser-)Schneiden eines rohrförmigen Halbzeugs erzeugt wird.

Mittlerweile gibt es auch erste Stents für Venen, insbesondere für Venen im Beinbereich. Bei derartigen Venenstents unterscheiden sich jedoch die Anforderungen zu herkömmlichen Stents, insbesondere Arterienstents, erheblich. Die Venen weisen nicht die Steifigkeit von Arterien auf, sind viel dünnwandiger und daher "weicher" und beweglicher. Daher müssen Venenstents in ihrer Festigkeit an die Schienungsnotwendigkeit dieser weichen Venenwandungen entsprechend angepasst sein. Andererseits muss eine Stützung in der Vene über einen wesentlich längeren Bereich als bei einer Arterie erfolgen können und der Venenstent sollte so elastisch sein, dass er sich einerseits an die Venen optimal anschmiegt und andererseits die Bewegung der Venen und deren Verlauf im Körper nicht beeinträchtigt.

Von der Firma Boston Scientific ist unter der Bezeichnung Wallstent® Venous Endoprothesis ein Venenstent bekannt, der aus einem Gitter ausgebildet ist, das aus Draht geflochten ist. Dieser Stent ist in Längen von 20 mm bis 90 mm bei Durchmessern von 10, 12, 14 und 16 mm in verschiedenen Kombinationen der beiden Maße verfügbar. Die einzelnen Stents werden aus einem langen, geflochtenen Drahtschlauch auf die gewünschte Länge zugeschnitten. An den Enden des Stents stehen somit spitze Drahtenden vor und die einzelnen Drähte sind frei gegeneinander verschiebbar, so dass die beiden Enden des Geflechts nicht stabil ausgebildet sind, sondern sich leicht verformen können.

Tanaka et al. (Conformity of Carotid Stents with Vascular Anatomy: Evaluation in Carotid Models; Am J Neuroradiol 25, 604-607 (2004)) berichten das Problem, dass die Konformität zwischen dem selbstexpandierenden Wallstent und der vaskulären Anatomie begrenzt ist. Durch den Mangel an Flexibilität werden Gefäßkrümmungen unerwünscht aufgerichtet und begradigt, welche in der Folge zu durch den Stent induzierten Knicken im weiteren Gefäßverlauf führen können.

Weiterhin wird von der Firma Cook Medical ein Stent mit der Bezeichnung Cook Zilver Vena Stent vertrieben, der in Venen eingesetzt wird. Dieser Stent wird durch Laserschneiden aus einem Metallrohr hergestellt und ist in Längen von 60, 100 und 140 mm bei Durchmessern von 14 bzw. 16 mm erhältlich. Die Radialkraft dieses lasergeschnittenen Stents ist höher als die von typischen arteriellen Stents, aber nicht optimal ausreichend für die Schienung von Venen. Zudem zeigt der Stent bei Krümmungen den typischen Effekt von lasergeschnittenen Stents, dass die einzelnen Streben an der inneren Seite der Krümmung in den Hohlraum hineinragen und damit in den Blutstrom, so dass ein erhöhtes Thrombogenitätsrisiko resultiert, und an der äußeren Seite der Krümmung in die Gefäßwand hineindrücken, was zu Läsionen und stärkeren Verletzungen führen kann, da sich Venenstents immer mit dem Blutgefäß in Bewegung befinden, insbesondere wenn der Venenstent im Bereich eines Gelenks oder z.B. der Hüfte positioniert werden muß.

Die Firma Optimed bietet seit kurzem den Optimed sinus-Venous Stent als Venenstent an. Dieser Stent ist in Längen von 60 bis 150 mm und jeweils mit Durchmessern von 12, 14, 16 und 18 mm erhältlich. Es handelt sich gleichfalls um einen lasergeschnittenen Stent, der aus einem Metallrohr durch Herausschneiden der Streben, die ein sehr aufwendiges und kompliziertes Muster aufweisen, hergestellt wird. Durch voneinander unabhängige Ring-Systeme der Streben, die wiederum untereinander mit sog. "Flash-Link"-Streben verbunden werden, wurde die Flexibilität dieses Stents gegenüber dem Cook Zilver Vena Stent verbessert.

Die Firma Veniti Medical hat jüngst die CE Kennzeichnung für ihr neu entwickeltes Veniti Vici Venous Stent System erhalten. Auch hierbei handelt es sich um lasergeschnittene Venenstents. Diese Stents sind in Längen von 60, 90 und 120 mm und jeweils Durchmessern von 12, 14 und 16 mm erhältlich. Das Streben-Design ist anders ausgeführt als bei den beiden Venenstents von Cook und Optimed. Genauso wie beim Optimed-Produkt soll das kompliziertere Streben-Design verbesserte Radialkraft und Flexibilität ermöglichen.

Die generellen Nachteile lasergeschnittener Stents, welche bauart- und prinzipbedingt immer die Form des Metallrohrs wieder einnehmen wollen, aus welchem Sie hergestellt worden sind, sind u.a. a) eine beschränkte Aufstellkraft (Radialkraft), b) eine begrenzte Flexibilität und Dauerbelastbarkeit an den Verbindungsstellen der Streben untereinander (resultierend daraus eine Bruchgefahr der Streben und damit des Stents) und c) daraus folgend ein hoher Aufwand, um Stents zu erhalten, die sich einigermaßen gut an die Venengefäßwand anlagern können. Da Venenstents mit z.B. den Gelenkbewegungen mitgehen müssen und die Venenwand wegen ihrer dünnen und schwachen Ausführung sehr leicht kollabiert, besteht ein erhöhtes Risiko des Abwanderns des Venenstents aus der ursprünglichen Position und damit eine Gefährdung für den Patienten. Die Radialkraft der lasergeschnittenen Stents ist zudem konstruktions- und herstellungsbedingt recht begrenzt, so dass sehr hoher Aufwand im Design der Stent-Streben getrieben werden muss, um eine verbesserte Radialkraft zu erreichen. Die Kombination der erforderlichen Radialkraft mit der erforderlichen Flexibilität und der optimalen Gefäßwandabdeckung ist bei einem lasergeschnittenen Stent nur mit sehr komplexen Streben-Designs erreichbar, aber auch das bisher nur in begrenztem Ausmaß. Die für Arterienstents entwickelten Streben-Designs sind mehr oder weniger ungeeignet für Venenstents, weil diese andere Anforderungen erfüllen müssen.

Die Anforderungen an Venenstents sind folglich in mehreren technischen und medizinischen Parametern wesentlich höher als an arterielle Stents, was sich in der sehr begrenzten Zahl bekannter dedizierter Venenstents im Vergleich zu der sehr großen Zahl bekannter arterieller Stents zeigt. Zudem ist außer dem nicht speziell als Venenstent entwickelten Wallstent® kein geflochtener Venenstent bekannt. Venenstents benötigen einen großen Durchmesser, da nicht nur eine generelle Durchgängigkeit des Gefäßes, sondern auch der erreichte Offenhaltungsdurchmesser von hoher medizinischer Bedeutung ist, um in distalen Venengefäßen den Normaldruck erreichen zu können.

Die im Stand der Technik bekannten geflochtenen endovaskulären Stents (also arteriellen Stents) weisen folgende Charakteristika auf:

| Eigenschaft | Vorteil | Nachteil |
|---|---|---|
| niedrige Anzahl von Überkreuzungspunkte bzw. runde Enden | schnelle, kostengünstige Herstellung (insbesondere bei Handflechtung) | schlechte Geflechtstabilität, mögliches Einschneiden in das Gewebe oder die Gefäßwand |
| große breite/breitgezogene Rauten ("querliegend") | Erhöhung der Radialkraft | hohe Elongation |
| niedrige Geflechtdichte | verbesserte Komprimierbarkeit | schlechte Geflechtstabilität |
| kürzere Ausführungen (keiner über 100mm) und kleine Durchmesser (keiner über 20mm) | schnelle, kostengünstige Herstellung (auch bei Handflechtung) | begrenzte Einsatzfähigkeit, bei längeren Strecken müssen mehrere Implantate hintereinander platziert werden mit allen damit verbundenen operativen und medizinischen Schwierigkeiten |

Interne Versuche haben gezeigt, dass nicht optimiert geflochtene Stents zumeist eine zu starke Elongation des Stents und eine ungenügende Radialkraft (Aufstellkraft) aufweisen. Daher liegen die Stents oft nur stellenweise an der Gefäßwand an und sind bei weichen Gefäßen nicht ausreichend wirksam. Seitlich neben dem Stentgeflecht verbleibende nicht abgestützte Bereiche bergen ein erhebliches Embolierisiko, weil kontinuierlich Blut durch das Geflecht strömt.

Geflochtene Stents sind bspw. aus der DE 198 43 822 A1, US 2009/0264985 A1, WO 2009/126244 A2, DE 197 50 971 A1 und WO 03/065934 A2 bekannt.

Die US 5,873,906 A zeigt einen zusammenfaltbaren Katheter, der im zusammengefalteten Zustand mittels eines Sackknotens zuasammengehalten wird. Der Sackknoten besitzt zwei Enden, wobei durch Ziehen an einem Ende der Sackknoten gelöst werden kann.

Aus der US 2004/0138734 A1 geht ein Implantat mit selbstexpandierenden Elementen hervor. Diese selbstexpandierenden Elemente werden zum Einsetzen des Implantats mittels schlaufenförmiger Gürtel zusammengehalten, wobei die Gürtel selbst durch Lösedrähte zusammengehalten werden, die sich durch die Schlaufen der Gürtel erstrecken. Werden die Lösedrähte aus den Schlaufen gezogen, dann lösen sich die Gürtel und die selbstexpandierenden Elemente können expandieren.

Aus der US 2007/0100427 A1 geht eine Vorrichtung hervor, mit welcher ein einmal gesetzter Stent im Blutgefäß wieder zusammengefaltet werden kann. Hierzu ist eine stabförmige Stütze permanent am Stent befestigt, in welcher Fäden zum Zusammenziehen des Stents geführt werden.

Aus der US 2012/0221093 A geht ein Kathetersystem zum Setzen eines Stents hervor, mit dem ein Stent an seinen beiden Enden gehalten und beim Setzen in das Blutgefäß unter Spannung gehalten wird.

Aus der US 2010/262157 A geht ein weiteres System zum Setzen eines Stents hervor, bei dem der Stent mittels eines Führungsstabes in komprimiertem Zustand in einen Katheter hineingeschoben wird. Der Stent ist mit einem Faden gesichert, der dann herausgezogen wird. Der Stent sitzt zum Setzen im Blutgefäß frei im Katheter und wird mit einem weiteren Schiebestab aus dem Katheter an der gewünschten Stelle herausgeschoben. Eine Änderung der Position des Stents im Blutgefäß ist nach dem Herausschieben aus dem Katheter nicht mehr möglich.

Ein weiteres System zum Setzen eines Stents ist in der US 2013/0006347 A beschrieben, das einen inneren und einen äußeren Katheter aufweist, wobei Fäden am inneren und äußeren Katheter derart befestigt sind, dass sie an den beiden Enden des Stents angreifen können und diesen unter Spannung setzen können.

Aus der US 7,097,653 geht eine Vorrichtung zum Setzen eines Implantates hervor, wobei das Implantat beim Setzen aktiv gestreckt wird.

In der WO 96/25124 A1 ist eine Prothesenvorrichtung zur Implantation in einem Körperhohlraum offenbart. Diese umfasst einen geflochtenen Stent, der aus mehreren Drähten geflochten ist. Der Stent umfasst einen zylindrischen Körperabschnitt, in dem erste und zweite Drähte sich in einem Basis-Schrägwinkel kreuzen sollen, und endseitig zwei im Wesentlichen zylindrische Endabschnitte, in denen die ersten und zweiten Drähte sich in einem Endabschnittsschrägwinkel kreuzen. Hierbei ist vorgesehen, dass der Endabschnittsschrägwinkel größer als der Basis-Schrägwinkel ist und somit eine größere Radialkraft im Endabschnitt als im Körperabschnitt erzeugt wird.

Aus der US 2014/0277573 A1 geht ein Stent zur Schienung einer Speiseröhre hervor. Dieser Stent weist an seinem distalen und seinem proximalen Ende aufgeweitete Befesti-gungsabschnitte auf, wobei zwischen den beiden endseitigen Befestigungsabschnitten ein zylindrischer Abschnitt vorgesehen ist. Der zylindrische Abschnitt bildet mehrere aneinander angrenzende Bereiche aus, die jeweils einen unterschiedlichen Komprimierungsgrad aufweisen. Dieser unterschiedliche Komprimierungsgrad wird durch unterschiedliche Winkel der den Stent ausbildenden Drähte erreicht.

Aus der US 2013/0090720 A1 geht ein selbstexpandierender Stent zur täglich wiederholten Benutzung und zur Behandlung von Krankheiten wie Schnarchen oder Schlafapnoe hervor, der vorzugweise in den Atemwegen anordbar ist. Dieser Stent umfasst eine distale funktionale Phase, eine proximale funktionale Phase und eine Übergangsphase, wobei die distale Phase eine größere Aufweitung als die proximale Phase aufweist und die Übergangsphase ebenso wie die proximale Phase aufgeweitet bzw. in etwa kegelförmig ausgebildet ist. Die distale Phase ist in etwa zylindrisch ausgebildet.

Aus der DE 10 2007 061 931 A1 geht ein Stent zur Behandlung eines Aneurysmas bzw. eine Erweiterung eines arteriellen Blutgefäßes hervor. Der Stent ist aus einer rohrförmigen Gitterstruktur ausgebildet, wobei an einem distalen und einem proximalen Ende Befestigungsabschnitte vorgesehen sind ,die im expandierten Zustand einen größeren Durchmesser aufweisen als der Abschnitt zwischen dem distalen und dem proximalen Abschnitt. Auf diese Weise soll es möglich sein das Implantat, mit den axial-äußeren Bereichen an gesunden Stellen eines Blutgefäßes zu fixieren, während im mittleren Bereich, also im Bereich eines Aneurysmas relativ wenig Druck ausgeübt wird, sodass die geschwächten Gefäßwände im Aneurysmabereich geschont werden. Auf diese Weise soll auch verhindert werden, dass sich der Stent in das Aneurysma hineinwölbt.

Aus der US 5,725,547 A1 geht ein Stent zur Stützung eines Körperlumens hervor. Dieser Stent ist in etwa rohrförmig ausgebildet und umfasst transversal ausgerichtete Abschnitte 24 und longitudinal ausgebildete Abschnitte 26, wobei die transversal ausgebildeten Abschnitte 24 Ausbuchtungen auf der Mantelwandung des Stents ausbilden. Die beiden transversal ausgebildeten Bereiche und die longitudinal ausgebildeten Bereiche, die alternierend angeordnet sind, sind aus dem gleichen Geflecht ausgebildet, das bedeutet die Geflechtstruktur ist identisch. Die Ausbuchtungen werden dabei durch eine mechanische Beaufschlagung der Stents in Verbindung mit einer Wärmebehandlung erzeugt. Auf diese Weise soll es möglich sein, ein Gefäß offenzuhalten und die entsprechenden Expansionskräfte zum Offenhalten des Gefäßes bzw. einer Arterie bereitzustellen. Eine durch die Drähte ausgebildete Mantelwan-. dung ist durch identischen Verlauf bzw. durch gleichartig ausgebildete Drähte ausgebildet, wobei die Verschiebungen in den transversalen und die unterschiedlichen Rauten in den transversalen und longitudinalen Abschnitten durch aufprägen der gewünschten Form und eine anschließende Wärmebehandlung erzeugt werden

Aus der EP 2 165 684 A1 geht ein in ein Blutgefäß mittels eines Mikrokatheters implantierbarer Stent hervor. Ein solcher Stent kann beispielsweise in etwa zylinderförmig ausgebildet sein, wobei Endabschnitte dichter geflochten sind als zwischen den Endabschnitten angeordnete Abschnitte. Hierbei können entlang der Längsrichtung des Stents auch zwischen den Endabschnitten mehrere Abschnitte vorgesehen sein, die eine unterschiedliche Flechtung aufweisen. Die unterschiedlich dicht geflochtenen Abschnitte sind hierbei insbesondere zum Halten des Stents auf einem entsprechenden Führungsdraht vorgesehen, um ein Verrutschen des Stents zu verhindern. Dieser Stent ist insbesondere zum Offenhalten eines verschlossenen Blutgefäßes vorgesehen, wobei dieses mittels Ballonexpansion beim Setzen des Stents geöffnet und durch die radialen Expansionskräfte des Stents offengehalten wird. Die unterschiedlich geflochtenen Abschnitte sind demgemäß vorgesehen, um den Stent besser auf einem Mikroführungsdraht anzuordnen, beim Einbringen zu halten und anschließend wieder entfernen zu können.

In der WO 2006/037086 A1 ist eine Einbringvorrichtung zum Setzen einer Stent-Anordnung in einem menschlichen Körper sowie eine entsprechende Stent-Anordnung hervor.

Aus der US 2005/0119722 A1 gehen eine rohrförmige Endoprothese und eine hohle Stützstruktur hervor.

Der Erfindung liegt die Aufgabe zugrunde, einen Stent zum Schienen einer Vene, mit welchem eine optimale Stützung von Venen möglich ist, und ein System zum Setzen eines Stents zu schaffen.

Die Aufgabe wird durch einen Stent mit den Merkmalen des Anspruchs 1 und durch ein System zum Setzen eines Stents mit den Merkmalen des Anspruchs 12 gelöst. Vorteilhafte Ausgestaltungen sind in den jeweiligen Unteransprüchen angegeben.

Ein erfindungsgemäßer Stent zum Schienen einer Vene besteht aus einem geflochten, rohrförmigen Stützkörper. Der Stent weist eine Länge von zumindest 60 mm auf. Der Stützkörper ist aus einem oder mehreren Drähten derart geflochten, dass Abschnitte des Drahtes bzw. der Drähte Rauten begrenzen.

Nach der vorliegenden Erfindung zeichnet sich ein Venenstent dadurch aus, dass er zumindest drei Versteifungsabschnitte aufweist, in welchen die Rauten in Längsrichtung kürzer als die Rauten der übrigen Abschnitte des Stents sind

Die Rauten der Versteifungsabschnitte können, aber müssen in Querrichtung nicht länger als die Rauten der übrigen Abschnitte sein. Die Versteifungsabschnitte sind mit dem gleichen Durchmesser wie die übrigen Abschnitte ausgebildet. In diesem Fall werden die Stränge beim Herstellen des Stents im Bereich der Versteifungsabschnitte durch einen größeren Flechtwinkel gegenüber der Längserstreckung des Stents als in den übrigen Abschnitten geflochten. Die Versteifungsabschnitte können auch einen größeren Durchmesser als die übrigen Abschnitte des Stents aufweisen (nicht beansprucht). In diesem Fall können die Versteifungsabschnitte auch genauso wie die oben erläuterten aufgeweiteten Bereiche durch Zusammendrücken bzw. Stauchen der entsprechenden Abschnitte hergestellt werden.

Unabhängig davon, wieviel Versteifungsabschnitte ausgebildet sind, weist ein solcher Stent besondere, vorteilhafte mechanische Eigenschaften auf. Die Versteifungsabschnitte verleihen dem Stent eine hohe radiale Steifigkeit. Die zwischen den Versteifungsabschnitten angeordneten weiteren Abschnitte, deren Rauten eine größere Längserstreckung als die Rauten der Versteifungsabschnitte aufweisen, sind hingegen weicher und flexibler. Sie verleihen daher dem Stent eine hohe Flexibilität bzw. Gelenkigkeit. Diese weiteren Abschnitte werden deshalb im Folgenden als Gelenkabschnitte bezeichnet. Die abwechselnde Anordnung von Versteifungsabschnitten und Gelenkabschnitten bildet somit eine Art Gliederkette aus den steifen, ringförmigen Versteifungsabschnitten und den biegsameren Gelenkabschnitten. Hierdurch kann sich ein solcher Venenstent an beliebige Kurven der Vene anpassen. Andererseits verhindern die Versteifungsabschnitte, dass die Vene kollabieren kann.

Insgesamt verleiht die Struktur eines solchen erfindungsgemäßen Stents aus alternierend Versteifungsabschnitten und Gelenkabschnitten diesem eine hohe Stabilität und verhindert eine starke Elongation der Gelenkabschnitte. Die Versteifungsabschnitte blockieren eine Interaktion der einzelnen Gelenkabschnitte miteinander, so dass die resultierende Elongation sich im Wesentlichen aus der Summe der Elongation der einzelnen Gelenkabschnitte ergibt. Die Elongation der einzelnen Gelenkabschnitte wird dadurch reduziert, dass die an den jeweiligen Versteifungsabschnitt angrenzenden Rauten des Gelenkabschnitts weniger beweglich sind als diejenigen in einem durchgehenden Geflecht mit der Rautenstruktur eines Gelenkabschnitts. Somit ist die Summe der Elongation der einzelnen Gelenkabschnitte geringer als die Elongation eines durchgehenden Geflechts mit der Rautenstruktur eines Gelenkabschnitts, welches in der Länge die Summe der Längen der einzelnen Gelenkabschnitte aufweist.

Der Stent weist zumindest drei Versteifungsabschnitte auf, wobei zwischen zwei benachbarten Versteifungsabschnitten ein Gelenkabschnitt angeordnet ist.

Da der Stent aus einem geflochtenen, rohrförmigen Stützkörper besteht, weist er keine Verzweigungen auf. Die Folge von abwechselnden Versteifungsabschnitten und Gelenkabschnitten ist somit in einem unverzweigten Stent ausgebildet.

Die Länge der Versteifungsabschnitte ist vorzugsweise kürzer als die Länge der Gelenkabschnitte.

Die Länge der Versteifungsabschnitte kann kleiner oder gleich 48 Rauten, 42 Rauten, 36 Rauten, 30 Rauten, 24 Rauten, 21, Rauten, 18 Rauten, 15 Rauten, zwölf Rauten, zehn Rauten, neun Rauten, acht Rauten, sieben Rauten, sechs Rauten oder fünf Rauten in Längsrichtung sein.

Die Länge der Versteifungsabschnitte umfasst vorzugsweise zumindest zwei Rauten, drei Rauten, vier Rauten, fünf Rauten oder zehn Rauten in Längsrichtung des Stents.

Die Länge der Gelenkabschnitte kann größer oder gleich als sechs Rauten, neun Rauten, zehn Rauten, elf Rauten, zwölf Rauten, 15 Rauten, 18 Rauten, 21 Rauten, 24 Rauten, 30 Rauten, 36 Rauten, 42 Rauten, 48 Rauten, 54 Rauten oder 60 Rauten in Längsrichtung sein.

Vorzugsweise sind die Rauten der einen oder mehreren Versteifungsabschnitte im unbelasteten Zustand des Stents in Längsrichtung um zumindest 10%, insbesondere zumindest 15% bzw. zumindest 30% kürzer als die Rauten der übrigen Abschnitte des Stents.

Dass der Stent zumindest drei Versteifungsabschnitte aufweist, in welchen die Rauten in Längsrichtung kürzer als die Rauten der übrigen Abschnitte des Stents sind, gilt vor allem für den unbelasteten Zustand des Stents. Im üblichen belasteten Zustand in einer Vene bleiben die Rauten der Versteifungsabschnitte kürzer als die Rauten der übrigen Abschnitte. Man kann den Stent jedoch derart zusammendrücken, dass auch die Versteifungsabschnitte derart elongieren, dass diese Beziehung nicht mehr aufrecht erhalten wird.

Da der Stent aus durchgehenden Strängen geflochten ist, die sich vom distalen zum proximalen Ende erstrecken, besitzt er eine homogene Geflecht-Struktur dahingehend, dass der Stent im Bereich eines jeden beliebigen Querschnitts die gleiche Anzahl an Strängen aufweist. Der Stent besitzt damit in axialer Richtung eine hohe Reißfestigkeit, die wesentlich höher ist als bei vergleichbaren Stents, die aus einem Rohr geschnitten sind. Bei derartigen Stents sind die Streben in flexiblen Abschnitten sehr dünn und durch das Schneiden aus einem Rohrabschnitt besitzen sie nicht wie runde Drähte eine geschlossene Materialstruktur, die wesentlich reißfester ist. Zudem werden Drähte bei der Herstellung durch Ziehen kaltverfestigt, was ihnen eine sehr hohe Reißfestigkeit verleiht. Die Gelenkabschnitte des erfindungsgemäßen Venenstents sind somit wesentlich stabiler als entsprechend flexible Abschnitte eines aus einem Rohrabschnitt lasergeschnittenen Stents.

Ein solcher Venenstent weist zumindest drei Versteifungsabschnitte auf. Insbesondere kann es zweckmäßig sein, in regelmäßigen Abständen von z.B. 2 bis 5 cm die Versteifungsabschnitte vorzusehen. Bei einem Stent mit einer Länge von 10 bis 30 cm können somit fünf bis 20 Versteifungsabschnitte vorgesehen werden.

Nach einem weiteren Aspekt der vorliegenden Erfindung zeichnet sich der Stent dadurch aus, dass im entspannten Zustand des Stents die Längserstreckung der meisten Rauten in Längsrichtung des Stents nicht kürzer als die Quererstreckung der Rauten ist. Dies gilt insbesondere für die Rauten der oben beschriebenen Gelenkabschnitte.

Erfindungsgemäß sind somit zumindest 60% bzw. 70% der Rauten, insbesondere zumindest 80% und vorzugsweise zumindest 90% der Rauten mit einer Längserstreckung ausgebildet, die im entspannten Zustand nicht kürzer und vorzugsweise sogar länger als die Quererstreckung ist.

Der Erfindung liegen die folgenden Erkenntnisse zu Grunde:

Venen sind ein Niederdrucksystem. Sie haben dünne Gefäßwände, sind daher sehr weich und kollabieren leicht. Sie haben folglich nicht eine so gerade Ausrichtung wie die Arterien, sondern weisen oft zahlreiche Krümmungen und Windungen auf. Venen haben einen größeren Anteil Bindegewebe und einen kleineren Anteil Muskelgewebe als Arterien. Durch diese dünnere stablisierende Muskelschicht der Vene entstehen völlig andere mechanische Anforderungen an Venenstents als an arterielle Stents, was eine andere Produktauslegung erfordert. Um eine ausreichende Stabilisierung von venösen Obstruktionen (Stenosen) zu erreichen, ist demzufolge bei Venenstents eine wesentlich höhere Radialkraft und gleichzeitig höhere Flexibilität erforderlich im Vergleich zu arteriellen Stents. Wenn der Stent nicht optimal an der Gefäßwand anliegt, besteht das Risiko, dass er wegen der relativ starken Bewegung der Venen (insbesondere in den Extremitäten) dislozieren, sich also unkontrolliert im Gefäß von der ursprünglichen Position wegbewegen kann.

Mit dem erfindungsgemäßen Stent werden die Nachteile von lasergeschnittenen Stents, die sich insbesondere beim Einsatz zur Schienung von Venen ausprägen, sowie die Nachteile eines einfachen, lediglich abgelängten geflochtenen Stents, der keine spezielle Anpassung an den gewünschten Einsatzzweck aufweist, überwunden.

Ein idealer Venenstent soll eine große Länge, eine ausreichend hohe Radialkraft und eine möglichst konsistente Radialkraftverteilung vom einen bis zum anderen Ende haben. Die hohe Radialkraft muss über das gesamte Leben des Patienten erhalten bleiben. Er muss flexibel sein, eine hohe Wandschlüssigkeit erzielen und im Gebrauch nach dem Einsetzen darf er keine Verkürzungstendenzen aufweisen. Außerdem muss er einfach absetzbar sein.

Bekannte geflochtene Stents besitzen Rauten, deren Quererstreckung größer als deren Längserstreckung ist. Die Rauten sind somit in Querrichtung gestreckt. Eine solche Rautenform verleiht dem Stent eine hohe Kompressionsfestigkeit, wodurch die Gefäße entsprechend stark gestützt werden können. Die Erfinder der vorliegenden Erfindung haben jedoch festgestellt, dass beim Komprimieren eines solchen Stents dieser sehr stark verlängert wird, das heißt, eine kleine Verringerung des Durchmessers durch Komprimieren des Stents führt zu einer großen Elongation des Stents. Sind hingegen die Rauten in Längsrichtung gestreckt, dann ist die Elongation des Stents beim Komprimieren wesentlich geringer. In Längsrichtung gestreckte Rauten führen dazu, dass die Kompressionsfestigkeit geringer als bei in Querrichtung gestreckten Rauten ist.

Da die Gefäßwandungen von Venen im Vergleich zu Arterien relativ weich sind, ist bei einem Venenstent eine hohe Kompressionsfestigkeit und gleichzeitig eine hohe Flexibilität erforderlich. Es hat sich gezeigt, dass die Verringerung der Kompressionsfestigkeit ausreichend kompensiert werden kann durch Vorsehen einer hohen Anzahl von Strängen und Verwendung eines entsprechend adaptierten Drahtdurchmessers. Hierdurch ist es möglich, einen an die Festigkeit der Gefäßwandungen der Venen optimal angepassten Venenstent zu schaffen. Bei längsgestreckten Rauten kann eine solche Anpassung an die Festigkeit sehr präzise erfolgen, ohne andere Eigenschaften des Stents wesentlich zu beeinträchtigen.

Venenstents unterscheiden sich von Arterienstents dadurch, dass sie wesentlich länger sind. Arterienstents besitzen meistens eine Länge von einigen wenigen Zentimetern. Venenstents werden zum Stützen von Venen über einen langen Abschnitt, der mindestens 6 cm lang ist und meistens sogar wesentlich länger, also mindestens 10 cm, insbesondere mindestens 15 cm bzw. mindestens 20 cm oder mindestens 25 cm ist. Wenn man derart lange geflochtene Venenstents komprimiert, dann führt die Kompression des Stents unweigerlich zu einer deutlichen bis starken Elongation des Stents. Die Erfinder haben festgestellt, dass bei längsgestreckten Rauten diese Elongation wesentlich geringer als bei quergestreckten Rauten ist. Aufgrund der großen Länge von Venenstents führt die Elongation beim Komprimieren des Venenstents im Absetzsystem und beim Absetzen im Blutgefäß zu einer unerwünschten erheblichen Elongation, die mit den längsgestreckten Rauten minimiert wird.

Vorzugsweise ist die Längserstreckung der Rauten im entspannten Zustand des Stents größer als die Quererstreckung. Die Längserstreckung ist insbesondere zumindest 5 % bzw. zumindest 10 % bzw. zumindest 15 % bzw. zumindest 20 % größer als die Quererstreckung. Je länger die Längserstreckung der Rauten relativ zur Quererstreckung ist, umso geringer ist die relative Elongation beim Komprimieren des Stents.

Es ist zweckmäßig, dass die Längserstreckung der Rauten im entspannten Zustand des Stents nicht länger als das 1,5-fache bzw. 1,4-fache bzw. 1,3-fache der Quererstreckung ist, damit eine ausreichende Kompressionsfestigkeit des Stents sichergestellt ist.

Ein Venenstent aus einem geflochtenen, rohrförmigen Stützkörper kann aus einem oder mehreren Drähten geflochten sein. Diese Drähte werden derart geflochten, dass sie sich in einem oder mehreren Strängen jeweils über die gesamte Länge des Stents erstrecken. Ein Strang ist somit ein Abschnitt eines Drahtes, der sich vom distalen zum proximalen Ende des Stents erstreckt.

Nach einem weiteren Aspekt der vorliegenden Erfindung zeichnet sich ein Venenstent dadurch aus, dass er aus einem oder mehreren Drähten derart geflochten ist, dass Abschnitte des Drahtes bzw. der Drähte Rauten begrenzen, und jeweils Abschnitte der Drähte, die sich über die gesamte Länge des Stents erstrecken als Stränge bezeichnet werden, wobei die Anzahl der Stränge n sich durch Multiplizieren eines Drahtdurchmessers $D_d$ mit einem Stentdurchmesser $D_s$ und einem Faktor F ergibt. Der sich hieraus ergebende rechnerische Wert ist auf eine ganze Zahl ab- bzw. aufzurunden. Der Faktor F ist eine Funktion in Abhängigkeit vom Drahtdurchmesser $D_d$. Der Faktor F unterliegt einer Toleranz von +/- 30 %. Insbesondere kann der Faktor F einer Toleranz von +/- 20 % und vorzugsweise von +/- 10 % unterliegen. Die Funktion des Faktors F kann durch folgende Spline-Funktionen dargestellt werden:

Polynom 1: (für $0,05 \leq D_d \leq 0,08$)

$$F = 91.64 + 44.35\, D_d - 20553.34 D_d^2 + 137022.25 D_d^3$$

Polynom 2: (für $0,08 < D_d \leq 0,1$)

$$F = 255.59 + -6103.93\, D_d + 56300.07\, D_d^2 - 183200.28\, D_d^3$$

Polynom 3: (für $0,1 < D_d \leq 0,12$)

$$F = 54.05 + -90.64\, D_d - 3668.35\, D_d^2 + 16694.46\, D_d^3$$

Polynom 4: (für $0,12 < D_d \leq 0,15$)

$$F = 113.30 + -1576.24\, D_d + 8730.26\, D_d^2 - 17746.13\, D_d^3$$

Polynom 5: (für $0,15 < D_d \leq 0,18$)

$$F = 38.95 + -79.93\, D_d - 1276.36\, D_d^2 + 4490.79\, D_d^3$$

Polynom 6: (für $0,18 < D_d \leq 0,2$)

$$F = 193.93 + -2660.39\, D_d + 13052.63\, D_d^2 - 22044.37\, D_d^3$$

Polynom 7: (für $0,2 < D_d \leq 0,22$)

$$F = -114.94 + 1969.54\, D_d - 10089.39\, D_d^2 + 16525.66\, D_d^3$$

Polynom 8: (für $0,22 < D_d \leq 0,25$)

$$F = 147.58 + -1609.72\, D_d + 6178.66\, D_d^2 - 8122.89\, D_d^3$$

Polynom 9: (für $0,25 < D_d \leq 0,3$)

$$F = 29.54 + -194.24\, D_d + 518.94\, D_d^2 - 576.60\, D_d^3$$

Polynom 10: (für $0,3 < D_d \leq 0,35$)

$$F = 110.08 + -910.08\, D_d + 2572.00\, D_d{}^2 - 2449.52\, D_d{}^3$$

Vorzugsweise ist der Draht aus Nitinol ausgebildet und der Drahtdurchmesser beträgt etwa 0,05 mm bis 0,35 mm. Ein aus dünnem Draht ausgebildeter Stent weist vorzugsweise mehr Stränge als ein aus dickem Draht ausgebildeter Stent auf.

Nach einer bevorzugten Ausbildung des Venenstents sind die Stränge zumindest an einem Ende des Stents paarweise miteinander verbunden, wobei diese paarweise Verbindung einen gebogenen Abschnitt bildet. Diese gebogenen Abschnitte stellen runde Enden dar. Die runden Enden stellen sicher, dass sich der Stent nicht in das Blutgefäß einschneidet, wodurch eine atraumatische Ausführung gegeben ist.

Vorzugsweise ist der Stent sowohl am proximalen als auch am distalen Ende mit runden Enden ausgebildet. Die runden Enden können durch Biegen eines langen Drahtes in mehrere Stränge erzeugt werden. Die Enden des Drahtes können auch paarweise miteinander verschweißt und/oder gecrimpt werden, sodass der Draht eine endlose Schleife bildet. Die Schweißnaht bzw. Crimpverbindung kann im Bereich der runden Enden als auch im Bereich der Stränge angeordnet sein.

Der Stent kann zumindest an einem Ende und vorzugsweise an beiden Enden paarweise verzwirbelte Stränge aufweisen. Hierdurch sind die Stränge paarweise aneinander fixiert, wodurch die Festigkeit des Stents in den Endbereichen erhöht wird, wenn keine runden Enden vorhanden sind.

An den paarweise verzwirbelten Enden der Stränge können Ösen ausgebildet sein, die einen ähnlich atraumatischen Abschluss wie die oben erläuterten runden Enden ausbilden.

Die verzwirbelten Enden der Stränge können auch verschweißt sein, so dass ein fester Zusammenhalt sichergestellt ist, ein atraumatischer Abschluß ausgebildet wird und dadurch gleichzeitig die Kompressionssteifigkeit in diesem Abschnitt des Stents erhöht wird, weil sich die Stränge nicht mehr gegeneinander verschieben können.

Der Stent kann auch einen Verzwirbelungsbereich aufweisen, in dem zwei oder mehrere Abschnitte des Drahtes bzw. der Drähte miteinander verzwirbelt sind. Der Verzwirbelungsbereich erstreckt sich vorzugsweise über einen kurzen Abschnitt in Längsrichtung des Stents und entlang des gesamten Umfangs des Stents. Dieser Verzwirbelungsbereich verhindert, dass sich die miteinander verzwirbelten Stränge in Axialrichtung zueinander bewegen. Es wird somit eine Axialfixierung der Stränge erzielt. Andererseits sind die verzwirbelten Abschnitte in Radialrichtung sehr flexibel. Ein solcher Verzwirbelungsbereich bewirkt somit eine axiale Entkopplung der durch den Verzwirbelungsbereich verbundenen Abschnitte des Stents.

Nach einer weiteren Weiterbildung des Stents kann dieser an einem oder an beiden Enden mit einem gegenüber dem übrigen Abschnitt des Stents aufgeweiteten (flared) Bereich ausgebildet sein. Dieser aufgeweitete Bereich dient dazu, den Stent in der Vene örtlich zu fixieren.

Der aufgeweitete Bereich erstreckt sich vorzugsweise über eine Länge von zumindest 5 mm. Die Aufweitung des Durchmessers des Stents durch den aufgeweiteten Bereich beträgt vorzugsweise zumindest 0,5 mm, insbesondere zumindest 1,0 mmund vorzugsweise zumindest 1,5 mm.

Im aufgeweiteten Bereich kann außerdem ein Versteifungsring vorgesehen sein. Dieser Versteifungsring kann durch querliegende Rauten ausgebildet sein. In diesem kurzen Abschnitt weisen die Rauten somit eine größere Quererstreckung als Längserstreckung im Vergleich zu den übrigen Abschnitten des Stents auf. Hierdurch besitzt der Stent im aufgeweiteten Bereich auch eine stärkere radiale Steifigkeit bzw. Kompressionsfestigkeit, wodurch die Versteifungswirkung zusätzlich unterstützt wird. Da die in Längsrichtung gestauchten Rauten nur über einen kurzen Bereich vorgesehen werden, ist die Erhöhung der Elongation bei einer Kompression des Stents gering.

Eine alternative Versteifung lässt sich einfach durch axiales Zusammendrücken des Stents herstellen. Nach dem Flechten eines aus Nitinoldraht bestehenden Stents wird dieser in seiner entspannten Stellung wärmebehandelt, wodurch sich die Materialstruktur im Draht so einstellt, dass der Stent die "entspannte Stellung" ohne interne Spannungen behält. Bei der Wärmebehandlung wird das Ende des Stents, an dem die Aufweitung vorgesehen werden soll, ein Stück in Längsrichtung zusammengedrückt. Hierdurch expandiert der Stent in diesem Bereich radial etwas und die Rauten werden in Längsrichtung zusammengedrückt. Die Wärmebehandlung des Stents kann hier in mehreren Teilschritten erfolgen, wobei beim ersten Schritt zunächst die Grundform des Stents eingeprägt wird, so dass das Geflecht stabilisiert wird, bevor in weiteren Schritten der bzw. die aufgeweiteten Bereiche durch eine zweite Wärmebehandlung strukturell fixiert werden.

Die oben erläuterten Aspekte können unabhängig voneinander ausgeführt sein. Es ist selbstverständlich auch möglich, Stents vorzusehen, bei welchen mehrere dieser unabhängigen Aspekte gemeinsam kombiniert sind.

Die erfindungsgemäßen Stents können auch über das Leistenband geführt werden, ohne dass sie brechen, was bei lasergeschnittenen Stents ein größeres Risiko ist. Die erfindungsgemäßen Stents besitzen eine hohe Lokationsstabilität, insbesondere wenn sie aufgeweitete Bereiche und/oder Verstärkungsabschnitte besitzen. Das Dislokationsrisiko aus der ursprünglichen Absetzposition ist daher im Vergleich zu bekannten Stents erheblich reduziert. Die Vena iliaca ist intolerant gegenüber nach der Stentung verbleibenden Läsionen. Daher sind lange Stents vorteilhaft. Die erfindungsgemäßen Stents können an sich mit beliebiger Länge hergestellt werden. Auch lange erfindungsgemäße Stents können mit atraumatischen Enden (runden Enden, Ösen) ausgebildet sein.

Ein weiterer Aspekt der vorliegenden Erfindung betrifft ein System zum Setzen eines Stents aus einem geflochtenen, rohrförmigen Stützkörper und insbesondere eines Stents zum Schienen einer Vene. Das System umfasst

- einen Implantationskatheter mit einem Volumen zum Aufnehmen des Stents im komprimierten Zustand,
- einen Positionierkatheter, der sich innerhalb des Implantationskatheters befindet und bündig am komprimierten Stent anliegen kann,
- einen flexiblen Ankerdraht, der durch Öffnungen am proximalen Ende des Stents geführt ist,
- einen Riegeldraht, der sich durch den Positionskatheter hindurch erstreckt und von zumindest einer Schlinge des Ankerdrahts umschlungen ist, sodass beim Entfernen des Riegeldrahtes der Ankerdraht frei wird und aus dem Stent und aus dem Positionierkatheter herausgezogen werden kann, wodurch der Stent freigegeben wird.

Beim Setzen eines Stents in ein Blutgefäß befindet sich der Stent zunächst im komprimierten Zustand im Implantationskatheter. Der Stent ist hierbei im Bereich des distalen Endes des Implantationskatheters angeordnet und liegt an der Innenfläche des Implantationskatheters an. Der Positionierkatheter schließt sich bündig am proximalen Ende des Stents an und erstreckt sich durch den Implantationskatheter hindurch bis zum proximalen Ende des Implantationskatheters. Der Ankerdraht ist am Stent eingehängt und mittels des Riegeldrahtes gesichert. Der Ankerdraht ist aus dem Positionierkatheter und Implantationskatheter herausgeführt. Hierdurch ist es möglich, mittels des Ankerdrahtes eine Zugkraft auf den Stent auszuüben.

Vorzugsweise weist der Positionierkatheter zumindest zwei und insbesondere drei sich über seine gesamte Längserstreckung erstreckende Kammern auf. Eine erste Kammer ist zur Durchführung des Riegeldrahtes und/oder des Ankerdrahtes und die zweite Kammer zur Durchführung eines Führungsdrahtes vorgesehen. Der Ankerdraht und der Riegeldraht können auch in zwei separaten Kammern geführt sein.

Dieses Kathetersystem wird in das Gefäß eines menschlichen oder tierischen Körpers eingeschoben. Das Kathetersystem wird so weit eingeschoben, bis der Katheter an der gewünschten Stelle angeordnet ist. Danach wird der Implantationskatheter ein Stück zurückgezogen, wodurch der Stent freigesetzt wird. In diesem Zustand ist es möglich, die Position des Stents nochmals zu verändern und den Implantationskatheter wieder über den Stent zu schieben, da der Stent mittels des Ankerdrahtes zurückgehalten werden kann. Somit steht erstmals ein System zum Absetzen eines geflochtenen Stents zur Verfügung, mit welchem eine vollständige Repositionierbarkeit - solange der Ankerdraht und der Riegeldraht nicht entfernt worden sind - auch dann gewährleistet ist, wenn der Stent bereits vollständig abgesetzt und expandiert ist. Bei bisher bekannten Systemen zum Absetzen eines geflochtenen Stents ist eine Repositionierbarkeit durch Wiederhereinziehen des Stents in das System nur solange möglich, wie ein wesentlicher Teil des Stents sich noch komprimiert im System befindet.

Ist der Stent an der richtigen Stelle positioniert, wird der Riegeldraht herausgezogen. Hierdurch wird der Ankerdraht freigegeben und kann aus dem Stent entfernt werden. Der Positionierkatheter und der Implantationskatheter werden vollständig aus dem Gefäß herausgezogen.

Nachdem der Implantationskatheter ein Stück zurück gezogen ist und der Ankerdraht freigegeben ist, werden der Riegeldraht und der Ankerdraht vollständig aus dem zu schienenden Gefäß entfernt, wobei es grundsätzlich egal ist, welches der beiden Elemente zuerst entfernt wird. Danach wird der Positionierkatheter aus dem Implantationskatheter und dem zu schienenden Gefäß und als letztes der Implantationskatheter entfernt. Der Führungsdraht kann dabei liegenbleiben, um nachfolgende weitere interventionelle Manöver durchzuführen. Der Führungsdraht kann aber auch zuerst entfernt werden, bevor der Stent abgesetzt wird, wenn dies gewünscht ist.

Wenn der Ankerdraht herausgezogen wird, bevor der Positionierkatheter herausgezogen wird, dann ist sichergestellt, dass der Stent nicht mit dem Ankerdraht zusammen ein Stück zurückgezogen wird.

Wenn zuerst der Positionierkatheter und dann der Implantationskatheter herausgezogen wird, wird nur einmal ein Katheter entlang der Innenfläche des zu schienenden Gefäßes gezogen und weniger Reibung an der Innenfläche des Gefäßes erzeugt.

Der oben erläuterte Stent und das System zum Setzen eines Stents sind zum Schienen einer Vene entwickelt und ausgebildet worden. Dieser Stent ist deshalb optimal an die Anforderungen einer zu schienenden Vene ausgebildet. Mit diesem Stent können jedoch auch andere Gefäße und/oder Hohlräume eines menschlichen oder tierischen Körpers geschient werden. Dies gilt insbesondere wenn die Schienung über einen längeren Abschnitt, als es bei herkömmlichen Arterienstents üblich ist, zu erfolgen hat.

Die Erfindung wird nachfolgend beispielhaft anhand der Zeichnungen näher erläutert. Die Zeichnungen zeigen in:

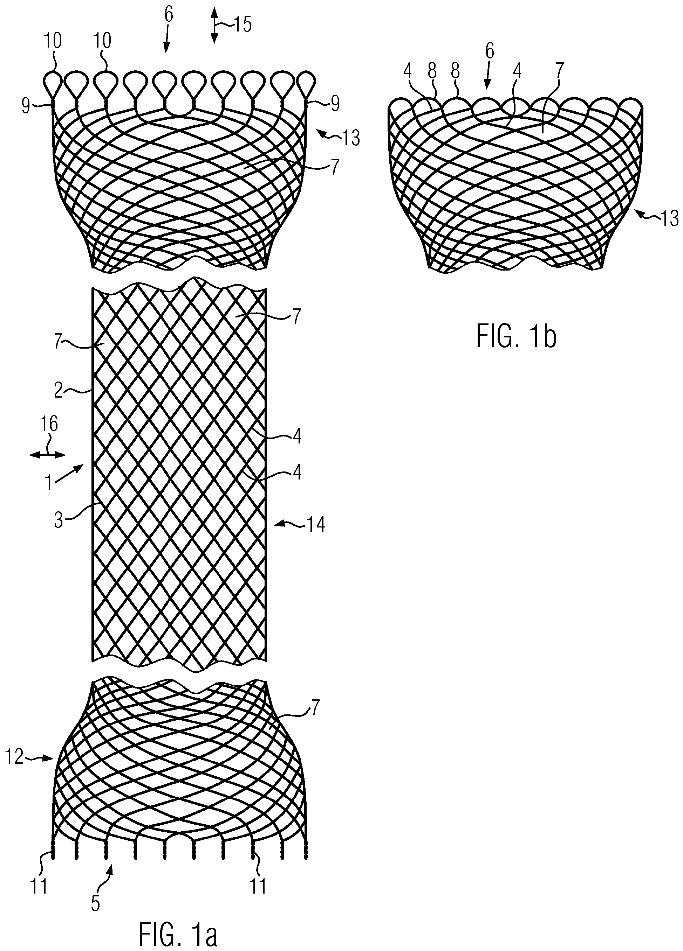

Fig. 1a          schematisch einen Venenstent in der Seitenansicht,

| | |
|---|---|
| Fig. 1b | einen Endbereich eines Venenstents mit sogenannten "runden Enden" schematisch in der Seitenansicht, |
| Fig. 2 | ein weiteres Ausführungsbeispiel eines Venenstents schematisch in einer Seitenansicht, |
| Fig. 3a - 3c | schematisch ein Positioniersystem für einen Venenstent und für andere Stents, wobei Teile des Stents und des Systems in perspektivischen Ansichten (Fig. 3a, 3c) und in einer Frontansicht (Fig. 3b; ohne Stent) dargestellt sind, und |
| Fig. 4 | das System zum Setzen eines Stents gemäß Fig. 3a - 3c in einer perspektivischen Ansicht, |
| Fig. 5 | ein weiteres Ausführungsbeispiel eines erfindungsgemäßen Venenstents schematisch in einer Seitenansicht, |
| Fig. 6 | eine Tabelle, welche wesentlichen Daten von Stents mit unterschiedlicher Drahtdicke und unterschiedlicher Anzahl von Strängen umfasst, |
| Fig. 7 | ein Diagramm eines Faktors F zur Berechnung der Anzahl der Stränge eines Stents in Abhängigkeit vom Drahtdurchmesser $D_d$, und |
| Fig. 8 | ein Diagramm, das den Durchmesser des Stents $D_d$ in Abhängigkeit von der Länge I von Rauten des Stents zeigt. |

Ein Stent 1 weist einen Stützkörper 2 zum Schienen einer Vene auf. Der Stent 1 kann ausschließlich aus dem Stützkörper 2 bestehen. Es ist jedoch auch möglich, dass am Stützkörper 2 weitere Funktionsteile, wie z.B. Filter oder dergleichen, angeordnet sind, welche keine Stützfunktion besitzen.

Der Stützkörper 2 ist aus einem oder mehreren Drähten 3 geflochten. Ein jeder der Drähte 3 bildet einen oder mehrere Stränge 4, wobei sich ein jeder Strang von einem proximalen Ende 5 des Stents 1 zu einem distalen Ende 6 des Stents 1 erstreckt. In dem hierdurch gebildeten Geflecht kreuzen sich die jeweiligen Stränge 4 paarweise, wobei durch die Stränge 4 jeweils Rauten 7 begrenzt werden.

Ein derart geflochtener Stent 1 weist im Vergleich zu herkömmlichen aus Rohrabschnitten geschnittenen Stents eine wesentlich höhere Biegsamkeit und Elastizität auf.

Die Drähte 3 bestehen vorzugsweise aus Metall, insbesondere einem Formgedächtnismetall. Das bevorzugte Material ist Nitinol. Grundsätzlich sind auch andere Materialien, wie Stahl oder steifer, insbesondere faserverstärkter Kunststoff möglich.

Der Stent ist vorzugsweise an einem Ende und insbesondere an beiden Enden mit sogenannten "runden Enden" 8 ausgebildet (Fig. 1b). Die runden Enden sind durch Biegen eines Drahtes 3 in mehrere Stränge 4 ausgebildet, wobei der Draht im Bereich der Biegung eine etwa kreissegmentförmige Form aufweist und nicht geknickt ist. Die Enden des Drahtes können auch paarweise miteinander verschweißt oder gecrimpt werden, so dass der Draht eine endlose Schleife bildet. Die Verbindungsstelle kann im Bereich der runden Enden 8 als auch im Bereich der Stränge 4 angeordnet sein. Durch Schweißen oder Crimpen können mehrere Drahtabschnitte zu einem einzigen Endlosdraht miteinander verbunden werden.

Bei dem in Fig. 1a gezeigten Ausführungsbeispiel ist der Stent 1 aus 16 Drähten 3 ausgebildet, die jeweils zu zwei Strängen 4 gebogen sind. Der Stent weist somit 32 Stränge 4 auf. Am distalen Ende 6 sind die beiden Stränge 4 eines jeden Drahtes 3 zu einem Verzwirbelungsabschnitt 9 verzwirbelt und bilden mit ihrem endseitigen Drahtabschnitt jeweils eine Öse 10 aus.

Die Ösen 10 bilden genauso wie die runden Enden 8 einen atraumatischen Abschluss.

Am proximalen Ende 5 sind die freien Enden zweier Stränge 4 miteinander zu einem weiteren Verzwirbelungsabschnitt 11 verzwirbelt. Am Verzwirbelungsabschnitt 11 können die Stränge 4 bzw. Drähte 3 miteinander verlötet sein.

Die Verzwirbelungsabschnitte 9 und 11 fixieren die Stränge 4 paarweise bezüglich der axialen Beweglichkeit. Hierdurch wird die Steifigkeit im Endbereich des Stents 1 erhöht.

Der Stent 1 weist sowohl am proximalen Ende 5 als auch am distalen Ende 6 jeweils einen aufgeweiteten Bereich 12, 13 auf. Ein sich zwischen den aufgeweiteten Bereichen 12, 13 erstreckender Hauptabschnitt 14 weist im entspannten Zustand einen im Wesentlichen konstanten Durchmesser auf. Der Durchmesser des Hauptabschnittes 14 im entspannten Zustand beträgt typischerweise 10 bis 20 mm. Die Rauten 7 des Hauptabschnittes 14 sind in Längsrichtung 15 nicht kürzer als in Querrichtung 16 und vorzugsweise sind sie in Längsrichtung 15 länger als in Querrichtung 16. Die Längserstreckung der Rauten ist im entspannten Zustand vorzugsweise zumindest 10 % und insbesondere zumindest 20 % länger als die Quererstreckung.

Der Durchmesser $D_s$ des Stents wird durch folgende Formel beschrieben:

$$D_S = \frac{n}{\pi}\sqrt{4 - l^2},$$

wobei n dieAnzahl der Rauten 7 des Stents entlang der Umfangsrichtung und I die Größe der Rauten in Längsrichtung 15 ist. Bei dieser Formel wird von Rauten ausgegenagen, die vier gleich große Seitenkanten aufweisen, wobei die Länge einer Seite auf "1" normiert ist, so dass mit dieser Formel der Durchmesser $D_s$ mit der "Längeneinheit einer Seitenkante" dargestellt wird.

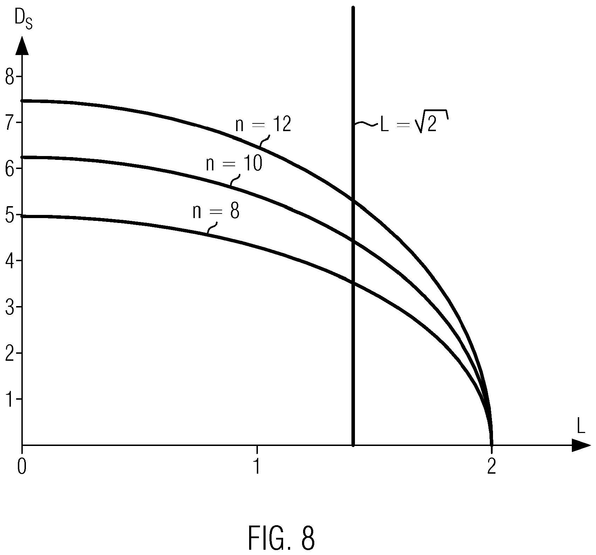

Figur 8 zeigt die Graphen dieser Funktion für n=12, n=10 bzw. n=8. Bei einer Länge I der Rauten von $\sqrt{2}$ (=1,4142) stimmt die Länge der Raute mit ihrer Höhe überein. Es ist dann eine quadratische Raute. Man erkennt anhand der Graphen, dass die Änderung der Länge der Rauten im Verhältnis zur Änderung des Durchmessers des Stents geringer ist, wenn die Rauten zumindest eine so große Länge wie Höhe aufweisen. Ist die Höhe hingegen größer, dann bewirkt eine kleine Änderung des Durchmessers des Stents eine große Längenänderung.

Ein geflochtener Stent 1, dessen meisten Rauten zumindest so lang wie hoch sind, wird beim Zusammendrücken wesentlich weniger verlängert als ein Stent, dessen meiste Rauten eine größere Quererstreckung als Längserstreckung aufweisen. Durch diese Ausbildung der Rauten wird sichergestellt, dass die gesamte Längenzunahme des Stents, auch wenn diese insgesamt sehr lang sind (z.B. zumindest 10 cm bzw. zumindest 20 cm bzw. zumindest 30 cm), relativ gering ist.

Vorzugsweise ist die Längserstreckung der Rauten 7 des Hauptabschnitts 14 im entspannten Zustand nicht länger als das 1,5-fache der Quererstreckung und vorzugsweise nicht länger als das 1,4-fache bzw. das 1,3-fache der Quererstreckung. Hierdurch wird sichergestellt, dass der Stent eine ausreichende Kompressionsfestigkeit aufweist. Die Kompressionsfestigkeit wird unter anderem von der Rautenform beeinflusst, wobei Stents mit sehr stark langgestreckten Rauten eine wesentlich geringere Kompressionsfestigkeit als Stents mit weniger gestreckten Rauten aufweisen. Die Kompressionsfestigkeit wird jedoch auch durch weitere Parameter, insbesondere die Anzahl der Stränge und die Dicke des verwendeten Drahtes beeinflusst.

Die aufgeweiteten Bereiche 12, 13 können u.a. durch axiales Zusammendrücken des jeweiligen Abschnittes des Stents erzeugt werden. Hierdurch werden die Rauten in Längsrichtung 15 ein Stück zusammengestaucht und in Querrichtung 16 gestreckt. Dies führt dazu, dass sich der Durchmesser des Stents 1 in den aufgeweiteten Bereichen 12, 13 gegenüber dem Hauptabschnitt 14 vergrößert. Zudem weisen die aufgeweiteten Bereiche 12, 13 aufgrund der in Querrichtung 16 stärker gestreckten Rauten 7 eine größere Kompressionsfestigkeit als der Hauptabschnitt 14 auf. Ein solcher Stent wird vorzugsweise aus einem Formgedächtnismaterial hergestellt, wobei nach dem Flechten des Stents dieser mit einer ersten Wärmebehandlung in seiner Form fixiert wird, wobei der Stent 1 mit einem konstanten Durchmesser über seine gesamte Länge gehalten wird, die dem Durchmesser des Stents im Bereich des Hauptabschnittes 14 entspricht. Durch diese Wärmebehandlung wird die Materialstruktur im Draht so eingestellt, dass der Stent diese Stellung ohne interne Spannungen beibehält. Danach werden die Endbereiche ein Stück zusammengedrückt, wodurch die aufgeweiteten Bereiche 12, 13 gebildet werden. In dieser Stellung wird der Stent ein zweites Mal wärmebehandelt, so dass die aufgeweiteten Bereiche 12, 13 entspannt werden und diese Form beibehalten. Aufgrund der stärkeren Quererstreckung der Rauten in den aufgeweiteten Bereichen 12, 13 wird hier eine größere relative Längserstreckung als im Hauptabschnitt 14 beim Komprimieren des Stents verursacht. Da diese aufgeweiteten Bereiche 12, 13 im Vergleich zum übrigen Stent sehr kurz sind (z.B. 5 mm bis 3 cm) ist die hierdurch verursachte Elongation gering und kann vernachlässigt werden.

Aufgrund der in Querrichtung 16 stärker gestreckten Rauten 7 weisen die aufgeweiteten Bereiche 12, 13 eine hohe radiale Festigkeit auf.

Die aufgeweiteten Bereiche 12, 13 dienen zum Fixieren der Position des Stents im Körpergefäß, insbesondere in einer Vene.

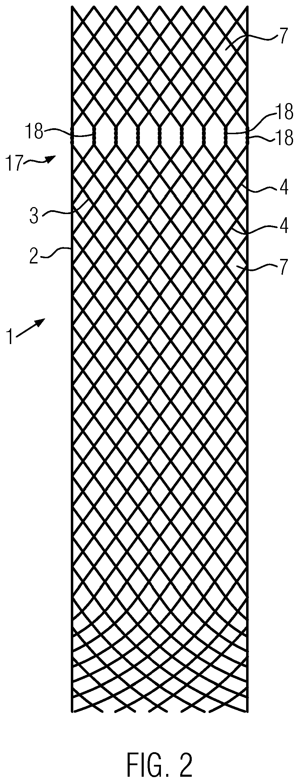

Der Stent kann auch mit einem Entkoppelungsabschnitt 17 ausgebildet sein, in dem jeweils zwei Stränge 4 zu weiteren Verzwirbelungsabschnitten 18 verzwirbelt sind (Fig. 2). Dieser Entkoppelungsabschnitt 17 entkoppelt die beiden an dem Entkoppelungsabschnitt 17 angrenzenden, geflochtenen Abschnitte in radialer Richtung, so dass die beiden geflochtenen Abschnitte mit unterschiedlichen Durchmessern ausgebildet sein können, ohne dass hierdurch Spannungen im Stent verursacht werden. Die Verzwirbelungsabschnitte 18 können nach radial außen oder nach radial innen verlaufen, ohne dass hierdurch Spannungen oder zumindest keine erheblichen Spannungen verursacht werden. Ein weiterer Effekt dieses Entkoppelungsabschnittes 17 ist, dass die Stränge 4 paarweise miteinander in Längsrichtung 15 fixiert sind. Hierdurch wird ein relatives Verschieben der Stränge 4 in Längsrichtung verhindert.

Ein Stent 1 kann keinen, einen oder mehrere derartige Entkoppelungsabschnitte 17 aufweisen.

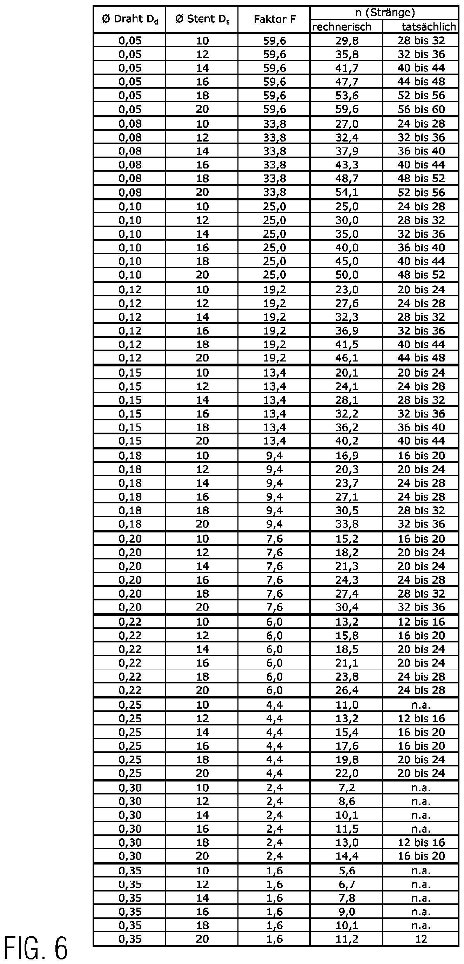

Der Stent 1 kann in unterschiedlichen Durchmessern (10 mm - 20 mm) mit unterschiedlichen Drahtstärken (0,05 mm - 0,35 mm) ausgebildet sein. In Abhängigkeit von der Drahtstärke bzw. Drahtdicke und dem Durchmesser des jeweiligen Stents ist der Stent mit einer unterschiedlichen Anzahl von Strängen 4 auszubilden. Die in Fig. 6 gezeigte Tabelle umfasst die Daten zum Drahtdurchmesser $D_d$, Stentdurchmesser $D_s$ und einen Faktor F zur Berechnung der Anzahl der Stränge eines Stents. Der Stent weist die gleiche Anzahl von Strängen mit der anderen Neigung auf, um jeweils durch zwei Paar Stränge mit unterschiedlichen Neigungen eine Raute 7 zu begrenzen. Die Anzahl der Stränge n ergibt sich durch Multiplizieren des Drahtdurchmessers $D_d$ mit dem Stentdurchmesser $D_s$ und dem Faktor F. Der sich hieraus ergebende rechnerische Wert ist entsprechend auf eine ganze Zahl ab- bzw. aufzurunden. In der Tabelle sind Bereiche mit der jeweils geeigneten Anzahl von Strängen n aufgeführt. Die tatsächlich geeignete Anzahl von Strängen variiert etwa ± 2 Stränge um den rechnerischen Wert. Im vorliegenden Ausführungsbeispiel bildet jeweils einer der Drähte 3 zwei Stränge 4 aus. Daher besitzen alle Stents 1 eine geradzahlige Anzahl von Strängen.

Je mehr Stränge vorgesehen werden, desto höher ist die Kompressionsfestigkeit des Stents. Es gibt jedoch noch weitere Einflüsse auf die Kompressionsfestigkeit des Stents wie z.B. die Rautenform und das tatsächlich verwendete Material des Drahtes. Die in Figur 6 gezeigten Daten gelten für Drähte aus Nitinol.

Der Faktor F kann in Abhängigkeit des Drahtdurchmessers $D_d$ durch die folgenden Spline-Funktionen dargestellt werden:

Polynom 1: (für $0,05 \leq D_d \leq 0,08$)

$$F = 91.64 + 44.35\,D_d - 20553.34 D_d^2 + 137022.25 D_d^3$$

Polynom 2: (für $0,08 < D_d \leq 0,1$)

$$F = 255.59 + -6103.93\,D_d + 56300.07\,D_d^2 - 183200.28\,D_d^3$$

Polynom 3: (für $0,1 < D_d \leq 0,12$)

$$F = 54.05 + -90.64\,D_d - 3668.35\,D_d^2 + 16694.46\,D_d^3$$

Polynom 4: (für $0,12 < D_d \leq 0,15$)

$$F = 113.30 + -1576.24\,D_d + 8730.26\,D_d^2 - 17746.13\,D_d^3$$

Polynom 5: (für $0,15 < D_d \leq 0,18$)

$$F = 38.95 + -79.93\,D_d - 1276.36\,D_d^2 + 4490.79\,D_d^3$$

Polynom 6: (für $0,18 < D_d \leq 0,2$)

$$F = 193.93 + -2660.39\,D_d + 13052.63\,D_d^2 - 22044.37\,D_d^3$$

Polynom 7: (für $0,2 < D_d \leq 0,22$)

$$F = -114.94 + 1969.54\,D_d - 10089.39\,D_d^2 + 16525.66\,D_d^3$$

Polynom 8: (für $0,22 < D_d \leq 0,25$)

$$F = 147.58 + -1609.72\,D_d + 6178.66\,D_d^2 - 8122.89\,D_d^3$$

Polynom 9: (für $0,25 < D_d \leq 0,3$)

$$F = 29.54 + -194.24\,D_d + 518.94\,D_d^2 - 576.60\,D_d^3$$

Polynom 10: (für $0,3 < D_d \leq 0,35$)

$$F = 110.08 + -910.08\,D_d + 2572.00\,D_d^2 - 2449.52\,D_d^3$$

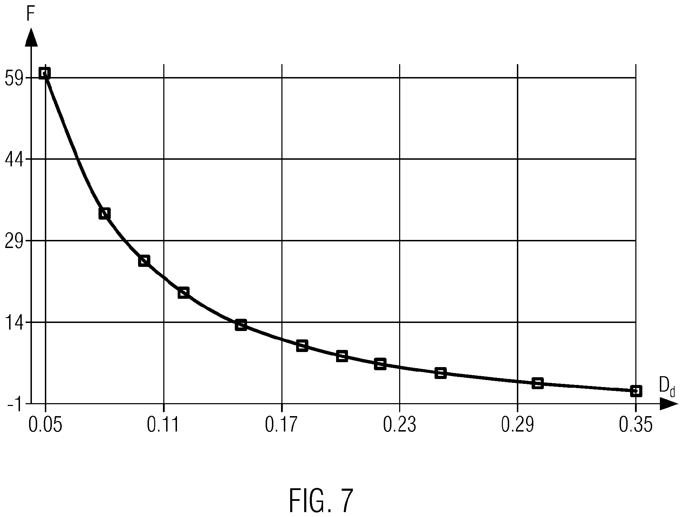

Die einzelnen Polynome 1 - 10 der Spline-Funktionen gelten auch unabhängig voneinander. Die Spline-Funktionen stellen den Bezug (siehe Figur 7) zwischen den Drahtdicken $D_d$ und den Faktoren F her, wie sie in der Tabelle in Figur 6 angegeben sind.

Die Konstante F ist ein empirischer Wert, der sich aus einer Vielzahl von Versuchen ergeben hat. Geeignete Stents können auch mit einer Toleranz des Faktors F von ± 30 %, insbesondere ± 20 % und vorzugsweise lediglich ± 10 % bzw. ± 5 % ausgebildet sein. Derartige Stents sind hinsichtlich ihrer Elongationseigenschaft und Kompressionsfestigkeit optimiert. Die Längserstreckung im Hauptabschnitt 14 des entspannten Stents ist mindestens genauso lang wie die Quererstreckung der Rauten. Vorzugsweise ist die Längserstreckung der Rauten 7 im Hauptabschnitt 14 länger als die Quererstreckung, jedoch nicht länger als das 1,5-fache der Quererstreckung. Dieser Stent ist vorzugsweise mit den aufgeweiteten Bereichen 12, 13 ausgebildet, welche eine gute Fixierung des Stents im Gefäß bewirkten.

Ein weiteres Ausführungsbeispiel eines erfindungsgemäßen Venenstents weist einen oder mehrere Versteifungsabschnitte 31 auf. In einem Versteifungsabschnitt sind die Rauten 7 in Längsrichtung kürzer als die Rauten der übrigen Abschnitte des Stents 1. Hierdurch sind die übrigen Abschnitte flexibler als die Versteifungsabschnitte. Diese übrigen Abschnitte werden im Folgenden als Gelenkabschnitte 32 bezeichnet.

Die Rauten 7 der Versteifungsabschnitte 31 können, aber müssen in Querrichtung 16 nicht länger als die Rauten der Gelenkabschnitte 32 sein.

Die Versteifungabschnitte 31 sind erfindungsgemäß mit dem gleichen Durchmesser wie die Gelenkabschnitte 32 ausgebildet. In diesem Fall werden die Stränge 4 beim Herstellen des Stents im Bereich der Versteifungsabschnitte 31 mit einem größeren Flechtwinkelbezüglich der Längserstreckung bzw. Längsrichtung 15 des Stents 1 als in den Gelenkabschnitten geflochten. Die Versteifungsabschnitte 31 können andererseits auch einen größeren Durchmesser als die übrigen Abschnitte des Stents 1 aufweisen (nicht beansprucht). In diesem Fall können die Versteifungsabschnitte 31, auch genauso wie die oben erläuterten aufgeweiteten Bereiche, durch Zusammendrücken bzw. Stauchen der entsprechenden Abschnitte in Längsrichtung hergestellt werden. An den distalen und proximalen Enden 5, 6 des Stents 1 können genauso wie bei den in Figur 1a und 1b dargestellten Ausführungsbeispielen runde Enden 8, Verzwirbelungsabschnitte 9 und/oder Ösen 10 mit oder ohne Verzwirbelungsabschnitten ausgebildet sein.

Die Versteifungsabschnitte verleihen dem Stent eine hohe radiale Steifigkeit. Die zwischen den Versteifungsabschnitten angeordneten Gelenkabschnitte, deren Rauten eine größere Längserstreckung als die Rauten der Versteifungsabschnitte 31 aufweisen, sind hingegen weicher und flexibler. Vorgesehen sind aufeinanderfolgend abwechselnd Versteifungsabschnitte 31 und Gelenkabschnitte 32. Die Gelenkabschnitte 32 sind typischerweise doppelt bis zehn mal so lang wie die Versteifungsabschnitte 31. Die abwechselnde Anordnung von Versteifungsabschnitten 31 und Gelenkabschnitten 32 bildet somit eine Art Gliederkette aus steifen, ringförmigen Versteifungsabschnitten 31 und den biegsameren Gelenkabschnitten 32. Ein solcher Venenstent 1 kann sich an beliebige Kurven der Vene anpassen. Die Versteifungsabschnitte 31 verhindern, dass die Vene kollabieren kann, und begrenzen die Elongation der Gelenkabschnitte.

Da der Stent aus durchgehenden Strängen geflochten ist, die sich vom distalen Ende zum proximalen Ende 6 erstrecken, besitzt er eine homogene Geflecht-Struktur. Der Stent besitzt damit in axialer Richtung eine hohe Reissfestigkeit, die wesentlich höher als bei vergleichbaren Stents ist, die aus einem Rohr lasergeschnitten sind.

Für einen solchen Venenstent mit Versteifungsabschnitten 31 und Gelenkabschnitten 32 gelten auch die in Figur 6 aufgeführten Daten zur Bestimmung der Anzahl n der Stränge 4.

Die Versteifungsabschnitte 31 weisen eine höhere Dichte an Strängen 4 als die Gelenkabschnitte 32 auf. In den Versteifungsabschnitten 31 sind die Stränge 4 mit einer stärkeren Neigung gegenüber der Längserstreckung des Stents 1 geflochten.

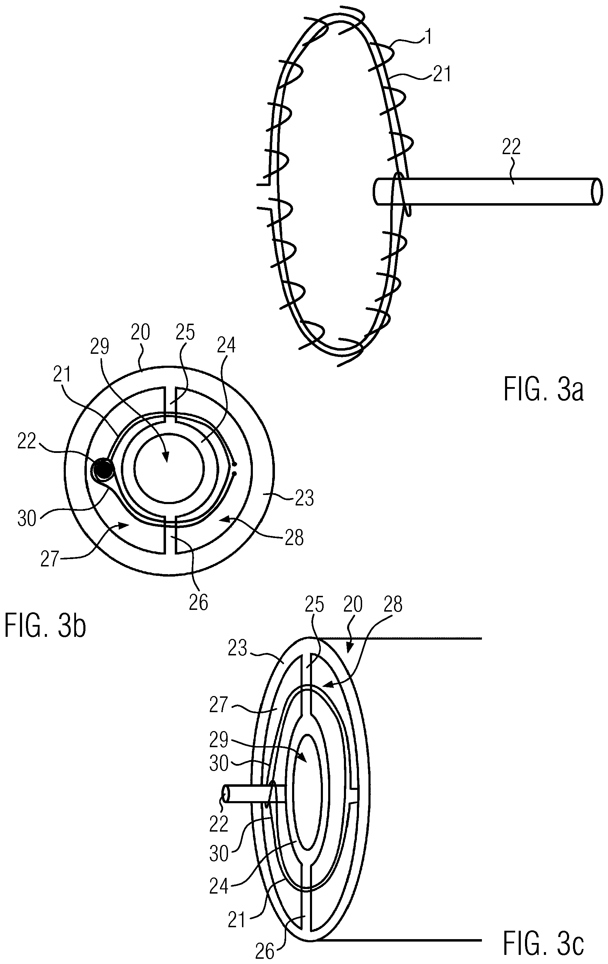

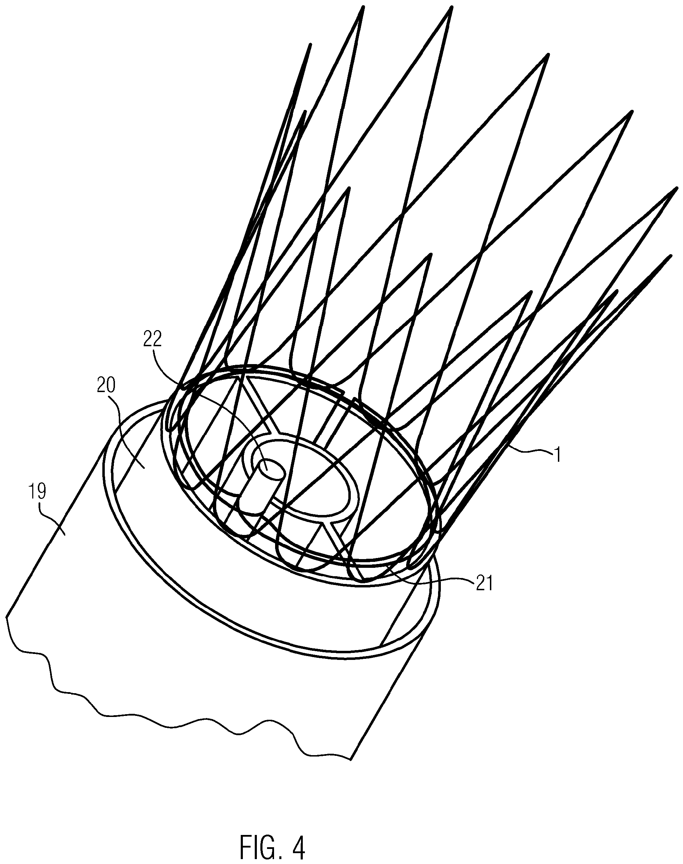

Nachfolgend wird anhand der Figuren 3a bis 4 ein System zum Setzen eines Stents erläutert.

Dieses System umfasst einen Implantationskatheter 19, einen Positionierkatheter 20, einen flexiblen Ankerdraht 21 und einen Riegeldraht 22. Der Positionierkatheter 20 ist im Implantationskatheter 19 verschieblich angeordnet, wobei die Außenfläche des Positionierkatheters 20 an der Innenfläche des Implantationskatheters 19 anliegt, wobei ein ausreichender Spalt dazwischen ausgebildet ist, um den Positionierkatheter 20 im Implantationskatheter 19 verschieben zu können. Der Implantationskatheter 19 ist zylinderförmig ausgebildet, der Positionierkatheter 20 hat eine zylinderförmige Außenwandung 23 und eine konzentrisch hierzu angeordnete zylinderförmige Innenwandung 24, wobei die Außenwandung 23 und die Innenwandung 24 mit zwei, vorzugsweise diametral gegenüberliegend angeordneten Stegen 25, 26 verbunden sind. Im Bereich zwischen der Innenwandung und der Außenwandung 23 sind somit zwei Kammern 27, 28 ausgebildet. Die Innenwandung 24 begrenzt eine weitere, im Querschnitt kreisförmige Kammer bzw. Lumen 29. Der Ankerdraht 21 ist durch die erste der beiden äußeren Kammern 27 geführt und erstreckt sich durch die Ösen bzw. Öffnungen des Geflechts im proximalen Endbereich des Stents 1 und ist mit endseitigen Schlaufen 30 aus dem Stent 1 nach innen zum Riegeldraht 22 geführt, der von den Schlaufen 30 des Ankerdrahtes 21 umschlungen ist. Der Riegeldraht erstreckt sich durch die zweite äußere Kammer 28. Sowohl der Riegeldraht 22 als auch der Ankerdraht 21 sind nach außerhalb des Positionierkatheters 20 und des Implantationskatheters 19 geführt und können von einem Operator betätigt werden. Durch Ziehen an demAnkerdraht 21 kann eine Zugkraft auf den Stent 1 ausgeübt werden, die in proximaler Richtung den Stent 1 zieht. Hierdurch kann bei einer Rückwärtsbewegung sichergestellt werden, dass der Stent 1 mit seinem proximalen Ende 5 am Positionierkatheter 20 anliegt. Wird der Riegeldraht 22 aus den Schlaufen 30 des Ankerdrahtes 21 gezogen, dann ist der Ankerdraht mit seinen Schlaufen 30 frei und kann aus den Ösen bzw. Öffnungen des Stents 1 herausgezogen werden. Hierdurch kann die Verbindung zwischen dem Ankerdraht 21 und dem Stent 1 gelöst und der Stent 1 entsprechend freigesetzt werden. Es können auch zwei oder mehrere Ankerdrähte vorgesehen sein.

Beim Setzen des Stents 1 in einem Blutgefäß befindet sich der Stent 1 zunächst im komprimierten Zustand im Implantationskatheter 19. Der Stent 1 ist hierbei im Bereich des distalen Endes des Implantationskatheters 19 angeordnet und liegt an der Innenfläche des Implantationskatheters 19 an. Der Positionierkatheter 20 schließt sich bündig an das proximale Ende des Stents 1 an und erstreckt sich durch den Implantationskatheter 19 hindurch bis zum proximalen Ende des Implantationskatheters 19. Der Ankerdraht 21 ist im Bereich seiner Schlaufen 30 mittels des Riegeldrahtes 22 gesichert. Der Ankerdraht 21 und der Riegeldraht 22 sind aus dem Positionierkatheter 20 und dem Implantationskatheter 19 an deren proximialen Enden herausgeführt. Diese Einheit, bestehend aus Implantationskatheter 19, Positionierkatheter 20, Ankerdraht 21, Riegeldraht 22 und Stent 1, wird in das Blutgefäß eingeschoben, bis der Stent an der richtigen Stelle positioniert ist.

Die innere Kammer bzw. das Lumen 29 des Positionierkatheters 20 dient zur Aufnahme eines Führungsdrahtes (nicht dargestellt), der zunächst bis zur gewünschten Position in das Blutgefäß eingeführt wird und zur Führung dieser Katheteranordnung beim Einführen in das Blutgefäß dient. Durch das Vorsehen separater Kammern 27, 28, 29 können sowohl der Führungsdraht, der Ankerdraht 21 und der Riegeldraht 22 getrennt voneinander durch den Positionierkatheter 20 geführt werden.

Ist diese Katheteranordnung soweit in das Blutgefäß eingeführt, dass sich der Stent an der gewünschten Position befindet, dann kann zunächst der Führungsdraht entfernt werden. Danach wird der Implantationskatheter 19 ein Stück zurückgezogen, wobei der Positionierkatheter 20 seine Position beibehält. Hierdurch wird sichergestellt, dass der Stent 1 nicht zusammen mit dem Implantationskatheter 19 zurückgezogen wird, da er an der Stirnseite des Positionierkatheters 20 anschlägt. Der Stent 1 wird aus dem Implantationskatheter 19 freigesetzt. Nach dem Freisetzen des Stents 1 wird vorzugsweise die Position des Stents 1 geprüft. Ist sie korrekt, dann wird der Riegeldraht 22 zurückgezogen und die Sicherung des Ankerdrahtes 21 gelöst. Der Ankerdraht kann dann aus dem Stent 1 herausgezogen werden, wodurch der Stent vollständig freigesetzt ist. Ist die Position des Stents 1 hingegen nicht korrekt, dann besteht die Möglichkeit, solange der Ankerdraht 21 gesichert ist, den Implantationskatheter 19 nochmals über den Stent 1 zu schieben und den Stent 1 neu zu positionieren.

Durch das Lumen 29 kann auch ein Ballonkatheter geführt werden, um insbesondere Abschnitte des Stents 1 zu expandieren. Dies kann insbesondere für die endseitigen aufgeweiteten Bereiche 12, 13 zweckmäßig sein, welche durch eine lokal begrenzte Dilatation in die Gefäße zurückgedrückt werden, um so den Stent zu immobilisieren.

Ist der Stent 1 korrekt gesetzt, dann werden zunächst der Ankerdraht 21, der Riegeldraht 22 und der Positionierkatheter 20 aus dem Blutgefäß vollständig herausgezogen. Als letztes wird dann der Implantationskatheter 19 herausgezogen.

Bezugszeichenliste

| 1 | Stent |
| 2 | Stützkörper |
| 3 | Draht |
| 4 | Strang |
| 5 | proximales Ende |
| 6 | distales Ende |
| 7 | Raute |
| 8 | rundes Ende |
| 9 | Verzwirbelungsabschnitt |
| 10 | Öse |
| 11 | Verzwirbelungsabschnitt |
| 12 | aufgeweiteter Bereich |
| 13 | aufgeweiteter Bereich |
| 14 | Hauptabschnitt |
| 15 | Längsrichtung |
| 16 | Querrichtung |
| 17 | Entkoppelungsabschnitt |

18  Verzwirbelungsabschnitt
19  Implantationskatheter
20  Positionierkatheter
21  Ankerdraht
22  Riegeldraht
23  Außenwandung
24  Innenwandung
25  Steg
26  Steg
27  erste Kammer
28  zweite Kammer
29  Kammer/Lumen
30  Schlaufe
31  Versteifungsabschnitt
32  Gelenkabschnitt

**Patentansprüche**

1.  Stent zum Schienen einer Vene, wobei der Stent (1) aus einem geflochtenen, rohrförmigen Stützkörper (2) besteht, der eine Länge von zumindest 60 mm aufweist, wobei der Stützkörper aus einem oder mehreren Drähten (3) derart geflochten ist, dass Abschnitte des Drahtes (3) bzw. der Drähte (3) Rauten (7) begrenzen, und der Stent zumindest einen Versteifungsabschnitt (31) und zumindest einen Gelenkabschnitt (32) aufweist, wobei die Rauten (7) im Versteifungsabschnitt (31) in Längsrichtung (15) kürzer als die Rauten (7) des Gelenkabschnitts (32) sind,
    **dadurch gekennzeichnet,**
    **dass** der Stent zumindest drei und vorzugsweise zumindest fünf Versteifungsabschnitte (31) aufweist, wobei zwischen zwei benachbarten Versteifungsabschnitten (31) zumindest ein Gelenkabschnitt (32) angeordnet ist und wobei die Versteifungsabschnitte (31) und die Gelenkabschnitte (32) einen im Wesentlichen gleichen Durchmesser aufweisen und die Versteifungsabschnitte (31) und die Gelenkabschnitte (32) abwechselnd angeordnet sind.

2.  Stent nach Anspruch 1,
    **dadurch gekennzeichnet,**
    **dass** der Versteifungsabschnitt (31) in Längsrichtung kürzer als der Gelenkabschnitt (32) ist.

3.  Stent nach einem der Ansprüch 1 bis 2,
    **dadurch gekennzeichnet,**
    **dass** die mehreren Versteifungsabschnitte (31) in regelmäßigen Abständen vorgesehen sind, wobei die Abschnitte vorzugsweise im Bereich von 2 bis 5 cm liegen.

4.  Stent nach einem der Ansprüche 1 bis 3,
    **dadurch gekennzeichnet,**
    **dass** die Drähte aus Nitinol ausgebildet sind und einen Drahtdurchmesser von 0,05 mm bis 0,35 mm aufweisen.

5.  Stent nach einem der Ansprüche 1 bis 4,
    **dadurch gekennzeichnet**
    **dass** zumindest an einem Ende und vorzugsweise an beiden Enden des Stents (1) die einzelnen Stränge (4) des zumindest einen Drahtes paarweise mit einem gebogenen Abschnitt verbunden sind, so dass sie jeweils ein rundes Ende (8) bilden.

6.  Stent nach einem der Ansprüche 1 bis 5,
    **dadurch gekennzeichnet,**
    **dass** zumindest an einem Ende und vorzugsweise an beiden Enden des Stents die Stränge (4) paarweise verzwirbelt sind.

7.  Stent nach Anspruch 6,
    **dadurch gekennzeichnet,**
    **dass** an den paarweise verzwirbelten Enden der Stränge (4) Ösen (10) ausgebildet sind und/oder die paarweise verzwirbelten Enden der Stränge (4) verschweißt sind.

8. Stent nach einem der Ansprüche 1 bis 7,
**dadurch gekennzeichnet,**
**dass** der Stent zumindest einen Verzwirbelungsbereich aufweist, in dem zwei oder mehrere Abschnitte des Drahtes bzw. der Drähte miteinander verzwirbelt sind, wobei der Verzwirbelungsbereich sich vorzugsweise über einen kurzen Abschnitt in Längsrichtung des Stents (1) und entlang des gesamten Umfangs des Stents (1) erstreckt.

9. Stent nach einem der Ansprüche 1 bis 8,
**dadurch gekennzeichnet**
**dass** der Stent an zumindest einem der beiden Enden, insbesondere am distalen Ende, und vorzugsweise an beiden Enden gegenüber dem übrigen Abschnitt des Stents einen aufgeweiteten (flared) Bereich aufweist.

10. Stent nach Anspruch 9,
**dadurch gekennzeichnet**
**dass** der aufgeweitete Bereich sich über eine Länge von zumindest 5 mm erstreckt und insbesondere eine Aufweitung des Durchmessers des Stents von zumindest 0,5 mm ausbildet.

11. Stent nach Anspruch 9 oder 10,
**dadurch gekennzeichnet**
**dass** im aufgeweiteten Bereich ein Versteifungsring ausgebildet ist, wobei der Versteifungsring Rauten vorzugsweise mit einer größeren Quererstreckung als Längserstreckung im Vergleich zu den übrigen Abschnitten des Stents aufweist.

12. System zum Setzen eines Stents zum Schienen einer Vene nach einem der Ansprüche 1 bis 11, wobei der Stent (1) aus einem geflochtenen, rohrförmigen Stützkörper (2) ausgebildet ist, umfassend

   - einen Implantationskatheter mit einem Lumen zum Aufnehmen des Stents im komprimierten Zustand,
   - einen Positionierkatheter, der sich innerhalb des Implantationskatheters befindet und bündig am komprimierten Stent anliegen kann,
   - einen flexiblen Ankerdraht, der durch Öffnungen des Stents geführt ist,
   - einen Riegeldraht, der sich durch den Positionierkatheter hindurch erstreckt und von zumindest einer Schlinge des Ankerdrahtes umschlungen ist, so dass beim Entfernen des Riegeldrahtes der Ankerdraht frei wird und aus dem Stent und aus dem Positionierkatheter heraus gezogen werden kann, wodurch der Stent freigegeben wird.

13. System nach Anspruch 12,
**dadurch gekennzeichnet,**
**dass** der Positionierkatheter zumindest zwei sich über die gesamte Länge des Positionierkatheters erstreckende Kammern aufweist, die zur Aufnahme eines Führungsdrahtes und zur Aufnahme des Riegeldrahtes bzw. des Ankerdrahtes ausgebildet sind.

14. System nach Anspruch 12 oder13,
**dadurch gekennzeichnet,**
**dass** die Öffnungen des Stents, durch welche der Ankerdraht geführt ist, am proximalen Endbereich des Stents angeordnet sind.

**Claims**

1. Stent for splinting a vein, whereas the stent (1) consists of a braided tubular supporting member (2) which has a length of at least 60 mm, whereas the supporting member is braided with one or more wires (3) in such a manner that sections of the wire (3) or the wires (3), resp., delimit rhombuses (7), and the stent comprises at least one reinforcement section (31) and at least one hinge section (32), whereas the rhombuses (7) in the reinforcement section (31) are shorter in longitudinal direction (15) than the rhombuses (7) in the hinge region (32),
**characterized in that**
the stent comprises at least three and preferably at least five reinforcement sections (31), whereas between two adjacent reinforcement sections (31) at least one hinge region (32) is provided, and wherein the reinforcement sections (31) and the hinge sections (32) have an substantially equal diameter and the reinforcement sections (31) and the hinge regions (32) are arranged alternately.

**2.** Stent according to claim 1,
**characterized in that**
the reinforcement section (31) is shorter in longitudinal direction than the hinge region (31)

**3.** Stent according to any one of claims 1 to 2,
**characterized in that**
the plurality of reinforcement sections (31) are provided at regular intervals, whereas the sections preferably are in the range of 2 to 5 cm.

**4.** Stent according to any one of claims 1 to 3,
**characterized in that**
the wires are made of nitinol and have a wire diameter of 0.05 mm to 0.35 mm.

**5.** Stent according to any one of claims 1 to 4,
**characterized in that**
at least at one end and preferably at both ends of the stent (1) the individual strands (4) of the at least one wire are connected in pairs with a bent section so that they each form a round end (8).

**6.** Stent according to any one of claims 1 to 5,
**characterized in that**
at least at one end and preferably at both ends of the stent the strands (4) are twisted in pairs.

**7.** Stent according to claim 6,
**characterized in that**
loops (10) are formed at the paired ends of the strands (4) and/or the paired twisted ends of the strands (4) are welded.

**8.** Stent according to any one of claims 1 to 7,
**characterized in that**
the stent has at least one twisted region in which two or more sections of the wire or the wires are twisted together, whereas the twisted region preferably extends over a short section in longitudinal direction of the stent (1) and along the entire circumference of the stent (1).

**9.** Stent according to any one of claims 1 to 8,
**characterized in that**
the stent has a flared region at least one of the two ends, in particular at the distal end, and preferably at both ends relative to the remaining section of the stent.

**10.** Stent according to claim 9,
**characterized in that**
the widened region extends over a length of at least 5 mm and in particular forms a widening of the diameter of the stent of at least 0.5 mm.

**11.** Stent according to claim 9 or 10,
**characterized in that**
a stiffening ring is provided in the widened region, whereas the stiffening ring has rhombuses preferably with a greater transverse extension than longitudinal extension compared to the remaining sections of the stent.

**12.** System for putting in place a stent for splinting a vein according to any one of claims 1 to 11, whereas the stent (1) is formed from a braided tubular supporting member (2), comprising

- an implantation catheter with a lumen for accommodating the stent in compressed state,
- a positioning catheter which is located inside the implantation catheter and can attach concisely to the compressed stent,
- a flexible anchor wire which is threaded through openings of the stent,
- a latch wire which extends across the positioning catheter and is embraced by at least one loop of the anchor wire so that during removal of the latch wire the anchor wire is released and can be pulled out of the stent and the positioning catheter, whereby the stent is released.

**13.** System according to claim 12,
**characterized in that**
the positioning catheter has at least two chambers extending over the entire length of the positioning catheter, which are provided for accommodating a guidewire and for accommodating the latch wire or the anchor wire, resp.

**14.** System according to claim 13,
**characterized in that**
the openings of the stent through which the anchor wire is threaded are arranged at the proximal end region of the stent.


**Revendications**

**1.** Stent destiné à servir de tuteur à une veine, sachant que le stent (1) est constitué d'un corps de soutien (2) tubulaire tressé qui présente une longueur d'au moins 60 mm, sachant que le corps de soutien est tressé à partir d'un ou de plusieurs fils (3) de telle manière que des sections du fil (3) ou des fils (3) délimitent des losanges (7), et le stent présente au moins une section de raidissement (31) et au moins une section articulée (32), sachant que les losanges (7) sont plus courts en direction longitudinale dans la section de raidissement (31) que les losanges (7) de la section articulée (32),
**caractérisé en ce que**
le stent présente au moins trois et de préférence au moins cinq sections de raidissement (31), sachant qu'au moins une section articulée (32) est disposée entre deux sections de raidissement (31) adjacentes et sachant que les sections de raidissement (31) et les sections articulées (32) présentent un diamètre sensiblement égal et les sections de raidissement (31) et les sections articulées (32) sont disposées en alternance.

**2.** Stent selon la revendication 1,
**caractérisé en ce que**
la section de raidissement (31) est plus courte en direction longitudinale que la section articulée (32).

**3.** Stent selon l'une des revendications 1 à 2,
**caractérisé en ce que**
les multiples sections de raidissement (31) sont prévues à intervalles réguliers, sachant que les sections sont situées de préférence dans une plage de 2 à 5 cm.

**4.** Stent selon l'une des revendications 1 à 3,
**caractérisé en ce que**
les fils sont constitués de Nitinol et présentent un diamètre de fil de 0,05 mm à 0,35 mm.

**5.** Stent selon l'une des revendications 1 à 4,
**caractérisé en ce que**
au moins à une extrémité et de préférence aux deux extrémités du stent (1), les brins (4) individuels de l'au moins un fil sont reliés par paire avec une section courbée de telle sorte qu'ils forment respectivement une extrémité (8) ronde.

**6.** Stent selon l'une des revendications 1 à 5,
**caractérisé en ce que**
au moins à une extrémité et de préférence aux deux extrémités du stent, les brins (4) sont entortillés par paire.

**7.** Stent selon la revendication 6,
**caractérisé en ce que**
aux extrémités des brins (4) entortillées par paire, des œillets (10) sont constitués et/ou les extrémités des brins (4) entortillées par paire sont soudées.

**8.** Stent selon l'une des revendications 1 à 7,
**caractérisé en ce que**
le stent présente au moins une zone d'entortillement dans laquelle deux ou plus de deux sections du fil ou des fils sont entortillées les unes avec les autres, sachant que la zone d'entortillement s'étend de préférence sur une section courte en direction longitudinale du stent (1) et le long de toute la circonférence du stent (1).

**9.** Stent selon l'une des revendications 1 à 8,
**caractérisé en ce que**
le stent présente, à au moins une des deux extrémités, en particulier à l'extrémité distale, et de préférence aux deux extrémités, une zone élargie (évasée) par rapport à la section restante du stent.

**10.** Stent selon la revendication 9,
**caractérisé en ce que**
la zone élargie s'étend sur une longueur d'au moins 5 mm et constitue en particulier un élargissement du diamètre du stent d'au moins 0,5 mm.

**11.** Stent selon la revendication 9 ou 10,
**caractérisé en ce que**
un anneau de raidissement est constitué dans la zone élargie, sachant que l'anneau de raidissement présente des losanges ayant de préférence une extension transversale plus grande qu'une extension longitudinale en comparaison avec les sections restantes du stent.

**12.** Système pour la pose d'un stent destiné à servir de tuteur à une veine selon l'une des revendications 1 à 11, sachant que le stent (1) est constitué d'un corps de soutien (2) tubulaire tressé, comprenant

  - un cathéter d'implantation avec une lumière pour l'admission du stent à l'état comprimé,
  - un cathéter de positionnement qui se trouve à l'intérieur du cathéter d'implantation et peut se placer en alignement contre le stent comprimé,
  - un fil d'ancrage flexible qui est guidé par des ouvertures du stent,
  - un fil-verrou qui s'étend à travers le cathéter de positionnement et autour duquel passe au moins une boucle du fil d'ancrage de telle sorte que, lors de l'enlèvement du fil-verrou, le fil d'ancrage devienne libre et puisse être retiré du stent et du cathéter de positionnement, ce qui a pour effet de libérer le stent.

**13.** Stent selon la revendication 12,
**caractérisé en ce que**
le cathéter de positionnement présente au moins deux chambres s'étendant sur toute la longueur du cathéter de positionnement, lesquelles sont constituées pour l'admission d'un fil de guidage et pour l'admission du fil-verrou ou du fil d'ancrage.

**14.** Stent selon la revendication 12 ou 13,
**caractérisé en ce que**
les ouvertures du stent par lesquelles le fil d'ancrage est guidé sont disposées au niveau de l'extrémité proximale du stent.

FIG. 1b

FIG. 1a

FIG. 2

FIG. 3a

FIG. 3b

FIG. 3c

FIG. 4

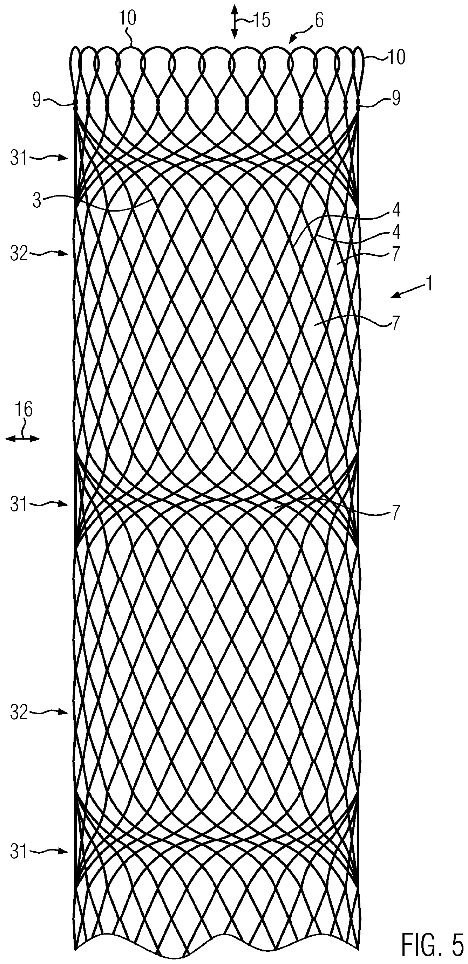

FIG. 5

| Ø Draht $D_d$ | Ø Stent $D_s$ | Faktor F | n (Stränge) | |
|---|---|---|---|---|
| | | | rechnerisch | tatsächlich |
| 0,05 | 10 | 59,6 | 29,8 | 28 bis 32 |
| 0,05 | 12 | 59,6 | 35,8 | 32 bis 36 |
| 0,05 | 14 | 59,6 | 41,7 | 40 bis 44 |
| 0,05 | 16 | 59,6 | 47,7 | 44 bis 48 |
| 0,05 | 18 | 59,6 | 53,6 | 52 bis 56 |
| 0,05 | 20 | 59,6 | 59,6 | 56 bis 60 |
| 0,08 | 10 | 33,8 | 27,0 | 24 bis 28 |
| 0,08 | 12 | 33,8 | 32,4 | 32 bis 36 |
| 0,08 | 14 | 33,8 | 37,9 | 36 bis 40 |
| 0,08 | 16 | 33,8 | 43,3 | 40 bis 44 |
| 0,08 | 18 | 33,8 | 48,7 | 48 bis 52 |
| 0,08 | 20 | 33,8 | 54,1 | 52 bis 56 |
| 0,10 | 10 | 25,0 | 25,0 | 24 bis 28 |
| 0,10 | 12 | 25,0 | 30,0 | 28 bis 32 |
| 0,10 | 14 | 25,0 | 35,0 | 32 bis 36 |
| 0,10 | 16 | 25,0 | 40,0 | 36 bis 40 |
| 0,10 | 18 | 25,0 | 45,0 | 40 bis 44 |
| 0,10 | 20 | 25,0 | 50,0 | 48 bis 52 |
| 0,12 | 10 | 19,2 | 23,0 | 20 bis 24 |
| 0,12 | 12 | 19,2 | 27,6 | 24 bis 28 |
| 0,12 | 14 | 19,2 | 32,3 | 28 bis 32 |
| 0,12 | 16 | 19,2 | 36,9 | 32 bis 36 |
| 0,12 | 18 | 19,2 | 41,5 | 40 bis 44 |
| 0,12 | 20 | 19,2 | 46,1 | 44 bis 48 |
| 0,15 | 10 | 13,4 | 20,1 | 20 bis 24 |
| 0,15 | 12 | 13,4 | 24,1 | 24 bis 28 |
| 0,15 | 14 | 13,4 | 28,1 | 28 bis 32 |
| 0,15 | 16 | 13,4 | 32,2 | 32 bis 36 |
| 0,15 | 18 | 13,4 | 36,2 | 36 bis 40 |
| 0,15 | 20 | 13,4 | 40,2 | 40 bis 44 |
| 0,18 | 10 | 9,4 | 16,9 | 16 bis 20 |
| 0,18 | 12 | 9,4 | 20,3 | 20 bis 24 |
| 0,18 | 14 | 9,4 | 23,7 | 24 bis 28 |
| 0,18 | 16 | 9,4 | 27,1 | 24 bis 28 |
| 0,18 | 18 | 9,4 | 30,5 | 28 bis 32 |
| 0,18 | 20 | 9,4 | 33,8 | 32 bis 36 |
| 0,20 | 10 | 7,6 | 15,2 | 16 bis 20 |
| 0,20 | 12 | 7,6 | 18,2 | 20 bis 24 |
| 0,20 | 14 | 7,6 | 21,3 | 20 bis 24 |
| 0,20 | 16 | 7,6 | 24,3 | 24 bis 28 |
| 0,20 | 18 | 7,6 | 27,4 | 28 bis 32 |
| 0,20 | 20 | 7,6 | 30,4 | 32 bis 36 |
| 0,22 | 10 | 6,0 | 13,2 | 12 bis 16 |
| 0,22 | 12 | 6,0 | 15,8 | 16 bis 20 |
| 0,22 | 14 | 6,0 | 18,5 | 20 bis 24 |
| 0,22 | 16 | 6,0 | 21,1 | 20 bis 24 |
| 0,22 | 18 | 6,0 | 23,8 | 24 bis 28 |
| 0,22 | 20 | 6,0 | 26,4 | 24 bis 28 |
| 0,25 | 10 | 4,4 | 11,0 | n.a. |
| 0,25 | 12 | 4,4 | 13,2 | 12 bis 16 |
| 0,25 | 14 | 4,4 | 15,4 | 16 bis 20 |
| 0,25 | 16 | 4,4 | 17,6 | 16 bis 20 |
| 0,25 | 18 | 4,4 | 19,8 | 20 bis 24 |
| 0,25 | 20 | 4,4 | 22,0 | 20 bis 24 |
| 0,30 | 10 | 2,4 | 7,2 | n.a. |
| 0,30 | 12 | 2,4 | 8,6 | n.a. |
| 0,30 | 14 | 2,4 | 10,1 | n.a. |
| 0,30 | 16 | 2,4 | 11,5 | n.a. |
| 0,30 | 18 | 2,4 | 13,0 | 12 bis 16 |
| 0,30 | 20 | 2,4 | 14,4 | 16 bis 20 |
| 0,35 | 10 | 1,6 | 5,6 | n.a. |
| 0,35 | 12 | 1,6 | 6,7 | n.a. |
| 0,35 | 14 | 1,6 | 7,8 | n.a. |
| 0,35 | 16 | 1,6 | 9,0 | n.a. |
| 0,35 | 18 | 1,6 | 10,1 | n.a. |
| 0,35 | 20 | 1,6 | 11,2 | 12 |

FIG. 6

FIG. 7

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19843822 A1
- US 20090264985 A1
- WO 2009126244 A2
- DE 19750971 A1
- WO 03065934 A2
- US 5873906 A
- US 20040138734 A1
- US 20070100427 A1
- US 20120221093 A
- US 2010262157 A
- US 20130006347 A
- US 7097653 B
- WO 9625124 A1
- US 20140277573 A1
- US 20130090720 A1
- DE 102007061931 A1
- US 5725547 A1
- EP 2165684 A1
- WO 2006037086 A1
- US 20050119722 A1

**In der Beschreibung aufgeführte Nicht-Patentliteratur**

- **TANAKA et al.** Conformity of Carotid Stents with Vascular Anatomy: Evaluation in Carotid Models. *Am J Neuroradiol,* 2004, vol. 25, 604-607